# EUROPÄISCHE PATENTSCHRIFT

(11) **EP 3 883 532 B1**
(45) Veröffentlichungstag und Bekanntmachung des Hinweises auf die Patenterteilung: **24.08.2022**
(21) Anmeldenummer: 19805704.4
(22) Anmeldetag: 22.11.2019
(51) Int. Cl.: A61K 8/25, A61K 8/34, A61K 8/44, A61K 8/46, A61Q 11/00, A61K 8/73, A61K 8/81

(54) **AUFSCHÄUMBARE ZAHNPFLEGEZUSAMMENSETZUNG, SYSTEM BESTEHEND AUS DER ZAHNPFLEGEZUSAMMENSETZUNG UND EINEM SPENDER SOWIE VERWENDUNG DER ZAHNPFLEGEZUSAMMENSETZUNG IN EINEM VERFAHREN ZUM GLEICHZEITIGEN REINIGEN MEHRERER, VORZUGSWEISE ALLER ZÄHNE**
LOW VISCOSITY DENTAL CARE COMPOSITION, SYSTEM CONSISTING OF DENTAL CARE COMPOSITION AND A DISPENSER AND USE OF THE DENTAL CARE COMPOSITION IN A METHOD FOR SIMULTANEOUSLY CLEANING A PLURALITY OF, PREFERABLY ALL TEETH
COMPOSITION DE SOINS DENTAIRES À FAIBLE VISCOSITÉ, SYSTÈME COMPRENANT LA COMPOSITION DE SOINS DENTAIRES ET UN DISTRIBUTEUR AINSI QU'UTILISATION DE LA COMPOSITION DE SOINS DENTAIRES DANS UN PROCÉDÉ DE NETTOYAGE SIMULTANÉ DE PLUSIEURS, DE PRÉFÉRENCE DE TOUTES LES DENTS

(30) Priorität: 23.11.2018 EP 18208042
(43) Veröffentlichungstag der Anmeldung: 29.09.2021
(73) Patentinhaber: BLBR GmbH, 82031 Grünwald (DE)
(72) Erfinder: KEINER, Michael, 35619 Braunfels (DE)
(74) Vertreter: Becker, Eberhard
(86) Internationale Anmeldenummer: PCT/EP2019/082325
(87) Internationale Veröffentlichungsnummer: WO 2020/104692

(56) Entgegenhaltungen:
- WO-A1-2014/098881
- WO-A2-01/62210
- DE-A1- 19 845 247
- US-A- 5 178 869
- US-A1- 2006 093 558
- UNKNOWN: "Amabrush - The 10 Second Toothbrush", Kickstarter , Juli 2017 (2017-07), XP002790981, Gefunden im Internet: URL:https://www.kickstarter.com/projects/a mabrush/amabrush-worlds-first-automatic-to othbrush/faqs [gefunden am 2019-05-02]

## Beschreibung

### GEBIET DER ERFINDUNG

Die vorliegende Erfindung betrifft eine aufschäumbare Mund-, Zahnpflege- und Zahnreinigungszusammensetzung, welche zur Anwendung bei der Zahnreinigung und -pflege mittels einer Zahnputzvorrichtung für die gleichzeitige Reinigung mehrerer, vorzugsweise aller Zähne eines Benutzers, besonders geeignet ist. Durch ihre niedrige Viskosität kann die Zusammensetzung gleichmäßig, schnell und effizient auf dem Mundeinsatz der Zahnputzvorrichtung aufgetragen und verteilt werden. Die erfindungsgemäße Zusammensetzung expandiert zu einem kurzzeitig stabilen Schaum mit guter Adhäsivität auf dem Mundeinsatz, der dann aber wieder verflüssigt werden kann. Die Handhabung wird dadurch wesentlich vereinfacht. Trotzdem wird eine sehr gute und schonende Reinigung der Zähne und des Zahnfleisches ermöglicht. Die vorliegende Erfindung betrifft weiterhin die Verwendung der aufschäumbaren Zusammensetzung in einem Verfahren zur gleichzeitigen Reinigung mehrerer, vorzugsweise aller Zähne mittels der Zahnputzvorrichtung. Außerdem stellt die vorliegende Erfindung ein System bereit, welches aus der erfindungsgemäßen Zahnpflege- und Zahnreinigungszusammensetzung und einem Spender besteht. Mit Hilfe dieses Systems kann die Zusammensetzung in der gewünschten Menge auf den Mundeinsatz appliziert werden und dort weiter zu einem Schaum expandieren.

### STAND DER TECHNIK

Generell liegen Mund- und Zahnpflegemittel in Pasten- oder Cremeform vor. Sie haben eine Viskosität von 60.000 bis 80.000 mPas (Millipascalsekunden; gemessen bei 20 °C) und werden aus Tuben oder Standbehältem durch manuelles Zusammendrücken des Behälters auf den Bürstenkopf einer Zahnbürste abgegeben. Die üblichen Anwendungsmengen liegen bei 1 bis 2 ml pro Benutzung. In selteneren Fällen liegen Mund- und Zahnpflegemittel in Gelform mit einer Viskosität von 10.000 bis 70.000 mPas (bei 20°C) vor. Auch hier wird eine Menge von 1 bis 2 ml durch Druck auf den Spenderbehälter auf den Bürstenkopf einer Zahnbürste appliziert. Eine Zahncreme mit relativ niedriger Viskosität - mit anderen Worten eine Liquid- oder Flüssigzahncreme - ist in der

EP 2 813 214 A1 offenbart. Diese Zusammensetzung wurde dahingehend formuliert, zwar eine stabile Einarbeitung von Aminfluoriden zu ermöglichen, aber trotzdem ausreichend viskos zu sein, um auf Bürstenköpfen herkömmlicher oder elektrischer Zahnbürsten appliziert werden zu können. Ein nach der Abgabe aus einem Bevorratungsbehälter aufschäumendes Zahnreinigungsmittel ist in der EP 1 257 250 B1 offenbart. Diese Zusammensetzung ist ebenfalls für die Anwendung mit herkömmlichen Zahnbürsten formuliert und weist deshalb eine Viskosität von mehr als 30.000 mPas auf.

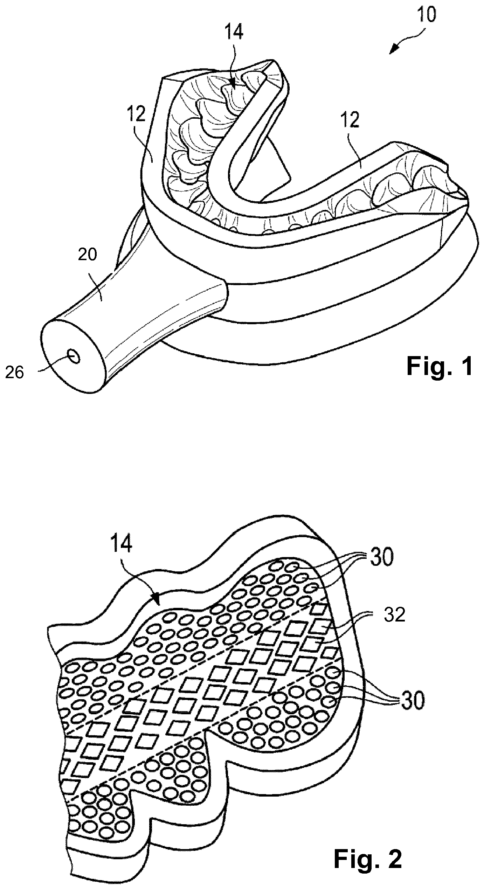

Die vorliegende Erfindung ist jedoch nicht auf die Anwendung der Mund- und Zahnpflege- und -reinigungszusammensetzung mit einer herkömmlichen Handzahnbürste oder einer üblichen elektrischen Zahnbürste gerichtet. Vielmehr gibt es neuere Entwicklungen auf dem Gebiet der Zahnreinigung, bei denen nicht mehr mit üblichen Bürstenköpfen gearbeitet wird, sondern mittels eines Mundeinsatzes gleichzeitig mehrerer oder alle Zähne des Benutzers gereinigt werden können. Diese Mundeinsätze (siehe z.B. Fig. 1) sind zumindest grob, vorzugsweise individuell, an die Gebiss- und Kieferform eines Benutzers angepasst und in der Lage, durch einen Motor zu Vibrationen angeregt, mehrere oder gar alle Zähne gleichzeitig zu reinigen. Entsprechende Zahnreinigungssysteme sind etwa in DE 10 2015 109891 A1, WO 2009/137671 A1 oder WO 2015/003681 A1 offenbart. Ein anderes solches System mit Mundeinsatz ist die Amabrush ("Amabrush - The 10 Second Toothbrush", Kickstarter, Juli 2017, URL:https://www.kickstarter.com/projects/amabrush/ amabrush-worlds-first-automatic-toothbrush/faqs).

Für die Benutzung in solchen Mundeinsätzen, die zum gleichzeitigen Reinigen mehrerer oder aller Zähne dienen, sind die im Stand der Technik bekannten Darreichungsformen von Zahnreinigungs- und -pflegeprodukten, wie beispielsweise Zahnpasten, Zahncremes oder Zahngele, aber auch die bekannten Schäume, ungeeignet. Die hochviskosen Produkte müssten mit einer Bürste oder einem Spatel auf den Reinigungsflächen der Mundeinsätze verteilt werden, wobei gerade die hohe Viskosität herkömmlicher Zahnpasten, Zahncremes oder -gele eine gleichmäßige Verteilung nahezu unmöglich macht.

Gleichzeitig würde die Menge der zu verwendenden Creme, Paste oder des Gels auf ca. 20 bis 25 ml pro Putzvorgang ansteigen, was zu einer übermäßigen Verschwendung von Ressourcen und einer Überdosierung führen würde. Eine Überdosierung kann unter anderem zu einem zu starken Dentinabrieb oder zu einer Schädigung des Zahnbeins entlang der Zahnfleischlinie führen.

Eine einfache Verdünnung herkömmlicher Zahnpflegeprodukte könnte zwar möglicherweise das Problem einer Überdosierung lösen, es löst aber nicht das Problem ein Produkt bereitzustellen, dass den anderen besonderen Erfordernissen bei der Verwendung eines der vorstehend beschriebenen Mundeinsätze Rechnung trägt. Eine verdünnte Zahncreme haftet beispielsweise nicht stabil genug an den Mundeinsätzen, insbesondere bei einer Überkopflage, und würde somit aus den Einsätzen herauslaufen, noch bevor diese in den Mund eingeführt werden können.

Auch die aus dem Stand der Technik bekannten Schäume erfüllen das Anforderungsprofil an eine solche Zahnreinigungszusammensetzung nicht. Ein Grund hierfür ist, dass die bekannten Schäume zu stabil sind und damit nicht ausreichend schnell zerfallen bzw. durch die Zähne des Benutzers zerdrückt werden können. Die hohe Schaumstabilität ist aus mehreren Gründen nachteilig. Zum einen quillt ein solcher stabiler Schaum beim Einsetzen der Mundeinsätze somit Großteils aus diesen heraus, was unerwünscht ist und auch einen Verlust an Aktivstoffen und damit einer verringerte Reinigungswirkung bedeutet. Zum anderen enthält der auf dem Mundeinsätzen verbleibende, also nicht durch die Zähne verdrängte, Schaum nach wie vor viele Luftblasen, welche die Übertragung der Vibrationen des Motors erschweren bzw. verringern, was letztlich die Reinigungsleistung negativ beeinflusst.

Es besteht daher Bedarf an neuartigen Zahnreinigungszusammensetzungen, welche zur Anwendung mit Zahnputzvorrichtungen zur gleichzeitigen Reinigung aller Zähne eines Benutzers geeignet sind, also den besonderen Erfordernissen bei der Verwendung solcher Zahnputzvorrichtungen Rechnung trägt. Die Zusammensetzungen müssen einfach und gleichmäßig auf entsprechenden Mundeinsätzen oder Mundeinsatzelementen zu applizieren sein und dort so ausreichend und stabil haften, dass sie nicht aus den Mundeinsätzen herauslaufen, bevor diese in den Mund eingeführt wurden. Gleichzeitig muss der Schaum aber auch wieder so instabil ausgestaltet sein, dass er nach dem Einsetzen der Mundeinsätze wieder zerfällt und/oder durch die Zähne zerdrückt werden kann. Darüber hinaus müssen die Formulierungen eine stabile Dispersion der Putzkörper in der Zusammensetzung gewährleisten. Nur so ist gewährleistet, dass diese gleichmäßig in den Mundeinsätzen verteilt und alle Zähne schonend und effizient gereinigt werden können. Auch müssen die Zusammensetzungen die sonstigen Anforderungen (Plaquereduzierung, Kariesprophylaxe, etc.) erfüllen, die an herkömmliche Zahnpasten gestellt werden.

Die herkömmlichen Verfahren zum Reinigen der Zähne mit Hand- oder Elektrozahnbürsten sind nicht nur zeitaufwendig, weil, bedingt durch die Dimension des Bürstenkopfs, die Zähne nacheinander gereinigt werden müssen, sie bergen auch das Risiko, dass entweder einzelne Zähne oder Gebissbereiche weniger gut gereinigt werden, weil sie bei der Reinigung vom Nutzer vergessen oder nicht lange genug gereinigt werden, oder dass einzelne Zähne oder Gebissbereiche zu lange und zu intensiv gereinigt und damit geschädigt werden. Verfahren zur gleichzeitigen Reinigung aller Zähne verbessern also den Erfolg der Reinigungs- und Pflegemaßnahme und erhöhen, insbesondere durch die Zeitersparnis, die Nutzer-Compliance.

Die vorstehend genannten Probleme wurden durch die vorliegende Erfindung gelöst.

### BESCHREIBUNG DER ERFINDUNG

Die vorliegende Erfindung betrifft gemäß einem ersten Aspekt eine Zahnpflege- und Zahnreinigungszusammensetzung zur Verwendung in einem Verfahren zum gleichzeitigen Reinigen mehrerer, vorzugsweise aller Zähne des Ober- und/oder Unterkiefers, mittels einer hierfür geeigneten Zahnputzvorrichtung, wobei die Zahnpflege- und Zahnreinigungszusammensetzung in aufgeschäumter Form auf einem Mundeinsatz oder mehreren Mundeinsätzen der Zahnputzvorrichtung dargereicht wird, und wobei die Zahnpflege- und Zahnreinigungszusammensetzung als wesentliche Komponenten
- 10 bis 40 Gew.-%, vorzugsweise 15 bis 30 Gew.-%, Glycerin,
- 10 bis 23,5 Gew.-% eines oder mehrerer gefällter Silikate, , vorzugsweise 10 bis 25 Gew.-% einer Mischung aus 2 bis 4 gefällten Silikaten,
- 0,05 bis 0,5 Gew.-% eines oder mehrerer Viskositätsverstärker,
- eine oder mehrere oberflächenaktive Substanzen, vorzugsweise mit einem Anteil von 0,2 bis 4,0 Gew.-% der einen oder der mehreren oberflächenaktiven Substanz(en), und
- mindestens 10 Gew.-% Wasser
umfasst.

Der Mundeinsatz oder die Mundeinsätze der Zahnputzvorrichtung ist/sind zumindest grob, vorzugsweise individuell, an die Gebiss- und Kieferform eines Benutzers angepasst und weist/weisen eine oder mehrere Aussparung zur Aufnahme der Zähne auf. Gemäß einer möglichen Ausführungsform ist der Mundeinsatz einteilig ausgebildet, das heißt der Mundeinsatz ist so ausgestaltet, dass er zur gleichzeitigen Aufnahme der Zähne des Ober- und der Zähne des Unterkiefers geeignet ist (siehe Fig. 1). Die Aussparungen zur Aufnahme der Gesamtheit der Zähne befinden sich also auf voneinander abgewandten Seiten des Mundeinsatzes bzw. öffnen sich in entgegengesetzte Richtungen. Alternativ kann die Zahnputzvorrichtung mehrere, vorzugsweise zwei Mundeinsätze enthalten, wobei der erste Mundeinsatz zur Aufnahme der Zähne des Oberkiefers und der zweite Mundeinsatz zur Aufnahme der Zähne des Unterkiefers ausgestaltet ist. Noch weiter alternativ kann die Zahnputzvorrichtung so ausgestaltet sein, dass sie nur einen Mundeinsatz aufweist, der zwar nur zur Aufnahme der Zähne eines Kiefers ausgestaltet ist, aber so flexibel gestaltet ist, dass er sich sowohl an die Zähne des Oberkiefers, als auch an die Zähne des Unterkiefers anpassen kann. In solch einem Fall würde zeitlich versetzt erst die Zahnreihe eines Kiefers und dann die Zahnreihe des anderen Kiefers gereinigt werden. Noch weiter alternativ kann der Mundeinsatz bzw. können die Mundeinsätze so ausgestaltet sein, dass sie jeweils zur Aufnahme von Zahnbereichen des Oberkiefers und/oder des Unterkiefers ausgestaltet sind, wobei die Zahnbereiche durch im Kiefer nebeneinanderliegenden Zähnen, beispielsweise den Backenzähnen oder den Schneidezähnen, gebildet werden. Zur gleichzeitigen Zahnreinigung werden dann gleichzeitig mehrere solcher Mundeinsätze verwendet, die so auszuwählen sind, dass alle Zähne des Ober- und/oder Unterkiefers aufgenommen werden. Alternativ könnte die Reinigung aller Zähne auch zeitlich versetzt erfolgen. Bevorzugte Ausgestaltungen der Mundeinsätze werden nachfolgend, auch unter Bezugnahme auf die Fig. 1 bis 5, noch im Detail beschrieben. All diese Mundeinsätze sind für die Darreichung der Zahnpflege- und Zahnreinigungszusammensetzung gemäß dem ersten Aspekt der Erfindung besonders geeignet.

Bevorzugte Ausführungsformen der erfindungsgemäßen Verwendung sind die Verwendung in einem Verfahren zur Vorbeugung und/oder Behandlung von Zahn- und Zahnfleischerkrankungen, insbesondere von Karies, Parodontose und Plaque, durch das gleichzeitige Reinigen mehrerer, vorzugsweise aller Zähne des Ober- und/oder Unterkiefers eines Benutzers.

Der Begriff "Zahnpflege- und Zahnreinigungszusammensetzung" wird hier und im Folgenden synonym mit dem Begriff "Formulierung" oder "Zusammensetzung" verwendet.

Im Sinne der vorliegenden Erfindung bedeutet die gleichzeitige Reinigung mehrerer Zähne, dass der Mundeinsatz bzw. die Mundeinsätze so ausgebildet ist/sind, dass vorzugsweise gleichzeitig mindestens 3, noch mehr bevorzugt mindestens 5, noch mehr bevorzugt mindestens 7 Zähne gereinigt werden können. Ganz besonders bevorzugt ist der Mundeinsatz bzw. sind die Mundeinsätze so ausgebildet, dass gleichzeitig alle Zähne des Oberkiefers und/oder des Unterkiefers gereinigt werden können. Gleichzeitig bedeutet, dass der Mundeinsatz oder die Mundeinsätze zu einem Zeitpunkt T1 mit allen zu reinigenden Zähnen in Kontakt stehen und diese reinigen. Im Unterschied dazu tritt der Bürstenkopf einer herkömmlichen manuellen oder elektrischen Zahnbüste zeitlich nacheinander mit den zu reinigenden Zähnen in Kontakt. Das erhöht nicht nur den Zeitaufwand beim Putzen, sondern birgt immer auch die Gefahr, dass nicht alle Zähne mit der gleichen Sorgfalt und Intensität gereinigt werden.

Die wesentlichen Komponenten der erfindungsgemäß verwendeten Zusammensetzung in den angegebenen Anteilsbereichen ermöglichen die Bereitstellung einer niedrigviskosen Zusammensetzung.

Eine wesentliche Komponente der Zusammensetzung ist Glycerin. Es war zwar im Stand der Technik grundsätzlich bekannt, dass Glycerin als ein mögliches Feuchthaltemittel in Zahnreinigungs- und -pflegeprodukten eingesetzt werden kann. Der einschlägige Stand der Technik hat dem Fachmann aber gleichzeitig eine große Palette an anderen möglichen Feuchthaltemitteln an die Hand gegeben. Feuchthaltemittel werden Zahnpflege- und - reinigungsprodukten üblicherweise zugesetzt, um ein Austrocknen der Zusammensetzung zu verhindern. Es wurde nun überraschend gefunden, dass Glycerin, wenn es in einem hohen Anteil eingesetzt wird, als Matrixbildner für die übrigen Inhaltsstoffe und als Stabilisator für die Zusammensetzung, insbesondere als Kurzzeit-Stabilisator für einen aus der Zusammensetzung erhaltenen Schaum, eingesetzt werden kann. Die vorteilhafte Mehrfacheignung von Glycerin war überraschend.

Ohne an diese Theorie gebunden wollen zu sein, erscheint es so, dass das Glycerin die Oberflächenspannung des in der Zusammensetzung ebenfalls mit einem relativ hohen Anteil enthaltenen Wassers senkt. Dies hat einerseits zur Folge, dass die Stabilität eines aus der Zusammensetzung erhaltenen Schaums dahingehend beeinflusst wird, dass für das Zeitfenster des Auftragens der Zusammensetzung auf den Mundeinsatz oder die Mundeinsätze eine hohe Schaumstabilität und Adhasion des Schaums erzielt werden kann. Dieses Zeitfenster liegt in der Praxis in der Regel in einem Bereich von wenigen Sekunden. Darüber hinaus bewirkt diese vorteilhafte Kombination der genannten Bestandteile aber auch, dass andererseits der gebildete Schaum nach dem Einführen der Mundeinsätze in den Mund der Schaum bei der Anwendung, also bei der Zahnpflege und Zahnreinigung, wieder so instabil wird, dass er ausreichend schnell wieder mehr oder weniger in eine flüssige Form zerfällt. Hierbei kann zusätzlich auch der mechanische Einfluss der Zähne förderlich sein, die den Schaum in den Aussparungen der Mundeinsätze beim Einsetzen desselben zusätzlich zerdrücken.

Weiterhin wurde überraschend gefunden, dass durch den Einsatz eines oder mehrerer gefällter Silikate die Zusammensetzung als solche, aber auch der aus der Zusammensetzung erhaltene Schaum vorteilhaft stabilisiert werden kann. Das oder die gefällten Silikate fungieren also nicht nur als Putzkörper, sondern auch als Stabilisator für die Dispersion und als Schaumstabilisator. Der so erhaltene Schaum ist nicht nur kurzzeitig stabil und wieder zerstörbar, sondern er zeichnet sich darüber hinaus auch durch seine gute Adhäsion auch in Kopfüberlage eines Mundeinsatzes aus.

Der Putzkörper einer Zahnpflege- und Zahnreinigungszusammensetzung, der auch als Abrasivmittel oder Poliermittel bezeichnet werden kann, ist im Grunde ein Schleifmittel und ermöglicht eine effektive Entfernung von Belägen beim Zähneputzen. Als Putzkörper werden in der Regel wasserunlösliche anorganische Stoffe eingesetzt, die den Zahnbeleg mechanisch entfernen, ohne den Zahnschmelz oder das Dentin zu schädigen. Wie auch bei den Feuchthaltemitteln hat der einschlägige Stand der Technik dem Fachmann eine sehr breite Palette an möglichen Putzkörpern an die Hand gegeben. Es war überraschend, dass durch die Auswahl von einem oder mehrerer gefällter Silikate als Putzkörper, insbesondere bei einem gleichzeitig hohen Anteil an Glycerin von mindestens 10 Gew.-%, vorzugsweise mindestens 15 Gew.-%, und einem Wassergehalt von mindestens 10 Gew.-% die Formulierungen als solche stabilisiert werden können und darüber hinaus auch kurzzeitig stabile, gut haftende Schäume bereitgestellt werden können.

Die Anwendung einer niedrigviskosen und aufschäumbaren Zusammensetzung ermöglicht das leichte und fehlerfreie Auftragen der Zusammensetzung auf einen Mundeinsatz, wo sie weiter zu einem Schaum expandieren kann. Die Expansion, also die Schaumbildung, kann durch den Einsatz eines oder mehrerer Treibgase bewerkstelligt werden. Hierfür wird die Zusammensetzung in einem geeigneten Behälter, vorzugsweise einer Aersoldose oder einer Bag-on-Valve Dose, bereitgestellt. Details hierzu werden nachfolgend beschrieben.

In einer bevorzugten Ausführungen des ersten Aspekts der vorliegenden Erfindung ist die Zusammensetzung so formuliert, dass die flüssige Vorformulierung, also die Zusammensetzung ohne Treibgas, eine Viskosität im Bereich von 200 - 30.000 m Pa s aufweist. Noch mehr bevorzugt liegt die Viskosität der Zusammensetzung ohne Treibgas in einem Bereich von 400 - 10.000 m Pa s und höchst bevorzugt bei weniger als 10.000 m Pa s. So ist beispielsweise eine Viskosität im Bereich von 500 - 9.000 m Pa s noch mehr bevorzugt. Noch weiter bevorzugt liegt die Viskosität in einem Bereich von 1.000 - 8.000 m Pa s. Der gewählte Viskositätsbereich stellt sicher, dass die Zusammensetzung gleichmäßig und einfach auf den Mundeinsatz oder die Mundeinsätze der Zahnputzvorrichtung appliziert werden kann und auch bis zur Schaumbildung genügend lange an diesem/diesen haftet.

Der Anteil des einen oder der mehreren oberflächenaktiven Substanzen an der Zusammensetzung beträgt vorzugsweise 0,35 bis 3,0 Gew-%, noch mehr bevorzugt 0,6 bis 2,5 Gew.-%. Besonders vorteilhafte oberflächenaktive Substanzen sowie bevorzugte gefällte Silikate, Viskositätsverstärker und weitere Bestandteile der Zusammensetzung werden nachfolgend im Zusammenhang mit der aufschäumbaren Zahnpflege- und Zahnreinigungszusammensetzung als solcher beschrieben. Diese Offenbarung soll ausdrücklich auch für den vorstehend beschriebenen ersten Aspekt der Erfindung gelten. Die vorliegende Erfindung betrifft gemäß einem zweiten Aspekt eine aufschäumbare Zahnpflege- und Zahnreinigungszusammensetzung als solche. Die Zusammensetzung gemäß dem zweiten Aspekt der vorliegenden Erfindung ist in vorteilhafterweise an die besonderen Erfordernisse bei der Verwendung in einem Mundeinsatz zur gleichzeitigen Reinigung mehrerer, vorzugsweise aller Zähne des Ober- und/oder Unterkiefers, gemäß dem ersten Aspekt der vorliegenden Erfindung angepasst.

Die erfindungsgemäße Zahnpflege- und Zahnreinigungszusammensetzung ist gemäß Anspruch 3, und umfasst als wesentliche Komponenten
- 15 bis 30 Gew.-%, vorzugsweise mindestens 20 Gew.-%, noch mehr bevorzugt mindestens 22 Gew.-%, Glycerin,
- optional wenigstens ein weiteres Feuchthaltemittel, neben Glycerin, wobei das wenigstens eine weitere Feuchthaltemittel vorzugsweise Sorbitol oder hydrogeniertes Stärkehydrolysat ist oder umfasst,
- 10 bis 23,5 Gew.-% eines Gemisches aus zwei oder mehr, vorzugsweise zwei bis vier, gefällten Silikaten,
- 0,2 bis 4,0 Gew.-% einer oberflächenaktiven Substanz oder mehrerer oberflächenaktiven Substanzen,
- 0,05 bis 0,5 Gew.-% eines Viskositätsverstärkers oder mehrerer Viskositätsverstärker, , und
- mindestens 10 Gew.-% Wasser.

Die Formulierung "wenigstens eines gefällten Silikats", bedeutet im Sinne der vorliegenden Erfindung, dass die erfindungsgemäße Zusammensetzung eine Silikatkomponente - im spezifizierten Anteilsbereich - enthält und diese Silikatkomponente entweder ein gefälltes Silikat/Silikat-Typ ist oder die Silikatkomponente ein Gemisch mehrerer gefällter Silikate/Silikat-Typen ist. Entsprechendes gilt für die Formulierungen "wenigstens ein weiteres Feuchthaltemittel", "wenigstens einer oberflächenaktiven Substanz", "wenigstens eines Viskositätsverstärkers", etc.

Die erfindungsgemäße Zusammensetzung gemäß dem zweiten Aspekt der vorliegenden Erfindung sowie die Zusammensetzung zur Verwendung gemäß dem ersten Aspekt der vorliegenden Erfindung enthält als wesentliche Komponente Glycerin und Wasser mit einem Anteil von mindestens 10 Gew.-%. Wie bereits im Zusammenhang mit dem ersten Aspekt der vorliegenden Erfindung erläutert, wurde überraschenderweise gefunden, dass in einer Zusammensetzung mit mindestens 10 Gew.-% Glycerin, das Glycerin nicht nur als Feuchthaltemittel fungiert, sondern in Kombination mit dem hohen Wasseranteil auch als Matrix für die übrigen Inhaltsstoffe und als Stabilisator für die Zusammensetzung, insbesondere als Kurzzeit-Stabilisator für einen aus der Zusammensetzung erhältlichen Schaum.

Die Komponente Glycerin ist deshalb in der erfindungsgemäßen Zusammensetzung mit einem Anteil von 15 bis 30 Gew.-%, bezogen auf die Gesamtzusammensetzung, umfasst. Vorzugsweise beträgt der Anteil an Glycerin an der Gesamtzusammensetzung mindestens 20 Gew.-%, noch mehr bevorzugt mindestens 22 Gew.-%. Die Obergrenze des Glycerinanteils beträgt vorzugsweise 28 Gew.-%. Erfindungsgemäß besonders bevorzugte Bereich sind beispielsweise 20 bis 30 Gew.-% oder 22 bis 28 Gew.-% Glycerin, bezogen auf die Gesamtzusammensetzung.

Neben den genannten Mengen bzw. Anteilen an Glycerin kann die erfindungsgemäße Zusammensetzung weitere Feuchthaltemittel enthalten. Das oder die weiteren Feuchthaltemittel dient bzw. dienen, wie das Glycerin, zum einen dazu, ein Austrocknen der Zusammensetzung zu verhindern. Das oder die weiteren Feuchthaltemittel können aber auch als Konsistenzgeber und als Kältestabilisator fungieren. Pharmazeutisch und/oder kosmetisch akzeptable Feuchthaltemittel sind dem Fachmann grundsätzlich bekannt. In einer erfindungsgemäß bevorzugten Ausführungsform umfasst die Zusammensetzung als weitere Feuchthaltemittel Sorbitol, hydrogeniertes Stärkehydrolysat, Xylit oder beliebige Mischungen von diesen bevorzugten Feuchthaltemitteln. Weitere, im Stand der Technik bekannte Feuchthaltemittel können zusätzlich in diesen bevorzugten Feuchthaltemitteln umfasst sein.

Der Anteil des oder der weiteren Feuchthaltemittel in Gew.-%, bezogen auf die Gesamtzusammensetzung, ist vorzugsweise so zu wählen, dass der Anteil an Glycerin plus des Anteils oder der Anteile der weiteren Feuchthaltemittel in Summe im Bereich von 30 bis 70 Gew.-% liegt. Vorzugsweise beträgt der Gesamtanteil 31,25 bis 65 Gew.-%, noch mehr bevorzugt 40 bis 62 Gew.-%, höchst bevorzugt 40 bis 60 Gew.-%, jeweils bezogen auf die Gesamtzusammensetzung.

Das in der erfindungsgemäßen Zusammensetzung enthaltene wenigstens eine gefällte Silikat fungiert, wie bereits vorstehend erwähnt, unter anderem als Putzkörper.

In den erfindungsgemäßen Zusammensetzungen kann der Putzkörper eine Substanz oder eine Mischung von verschiedenen Substanzen enthalten.

Die erfindungsgemäßen Zusammensetzungen enthalten als Putzkörper wenigstens eine Verbindung aus der Gruppe der Silikate, die im einschlägigen Stand der Technik als gefällte Silikate ("hydrated Silica") bezeichnet werden. Geeignete gefällte Silikate sind im Handel erhältlich und dem Fachmann bekannt.

Die gefällten Silikate weisen bevorzugt eine mittlere Teilchengröße im Bereich von 1 bis 50 µm, noch mehr bevorzugt von 6 bis 14 µm.

Weiterhin weisen die gefällten Silikate bevorzugt eine Einlehner Abrasion im Bereich von kleiner 2,0 bis 30 mg Verlust / 100 000 Umdrehungen auf. Im Handel sind gefällte Silikate erhältlich, die sich in ihrer mittleren Teilchengröße und in ihrer Abrasivität unterscheiden. Erfindungsgemäß besonders geeignete gefällte Silikate sind beispielsweise unter der Bezeichnung ZEODENT^{®}103, ZEODENT^{®}115, ZEODENT^{®}165 und ZEODENT^{®}167 erhältlich. Während ZEODENT^{®}103 relativ stark abrasiv wirkt und im Vergleich zu den anderen genannten ZEODENT-Formen die geringste mittlere Teilchengröße aufweist, hat ZEODENT^{®}115 einen deutlich geringeren Abrasionswert.

Noch geringer ist der Abrasionswert von ZEODENT^{®}165 und ZEODENT^{®}167, diese weisen aber eine höhere mittlere Teilchengröße auf. Im Umfang der vorliegenden Erfindung sind ebenso beliebige Mischungen von diesen beispielhaft genannten gefällten Silikaten umfasst.

Ein Ziel des Einsatzes von gefällten Silikaten oder einer beliebigen Mischung von solchen ist es einen mittleren bis hohen Abrasionsfaktor der Zahnpflege- und Zahnreinigungszusammensetzung bereitzustellen.

Der Abrasionsfaktor der Zusammensetzung wird vorzugsweise als RDA-Wert der Zusammensetzung angegeben. "RDA" steht für radioaktiver Dentinabrieb. Der RDA-Wert der erfindungsgemäßen Zusammensetzungen liegt bevorzugt in einem Bereich von 30 bis 160. Bei einem RDA-Wert von weniger als 30 sind die Reinigungseffekte der Zusammensetzung geringer, während bei einem RDA-Wert von mehr als 160 die Gefahr besteht, dass die Zusammensetzung so abrasiv ist, dass sie das Zahnbein entlang der Zahnfleischlinie beschädigen kann. Besonders bevorzugt liegt der RDA-Wert der erfindungsgemäßen Zusammensetzung in einem Bereich von 50 bis 130. In diesem Bereich ist die erzielte schonende Reinigungswirkung besonders vorteilhaft. Der Abrasionsfaktor und insbesondere der RDA-Wert einer Zahnreinigungszusammensetzung hängt sowohl von der Abrasivität der verwendeten Putzkörper als auch von deren Konzentration in der Zusammensetzung ab. Deshalb werden die gefällten Silikate in einer ausreichenden Menge und gegebenenfalls in einem geeigneten Mischungsverhältnis zugesetzt, um den gewünschten Abrasionsfaktor einzustellen.

Die in der erfindungsgemäßen Zusammensetzung umfassten gefällten Silikate fungieren aber nicht nur als Putzkörper, sondern sie haben darüber hinaus, zumindest zum Teil auch verdickende Eigenschaften. In einer bevorzugten Ausführungsform der vorliegenden Erfindung ist die Menge an verdickenden gefällten Silikaten mindestens 1 Gew.-%, vorzugsweise mindestens 1,5 Gew.-%, noch mehr bevorzugt mindestens 3 Gew.-%, bezogen auf die Gesamtzusammensetzung. Die Obergrenze an solchen verdickenden gefällten Silikaten beträgt 10 Gew.-%, vorzugsweise 7 Gew.-%, noch mehr bevorzugt 3,5 Gew.-% oder 3 Gew.-%.

Das wenigstens eine gefällte Silikat ist mit einem Anteil von 10 bis 23,5 Gew.-%, bezogen auf die Gesamtzusammensetzung, in der erfindungsgemäßen Zusammensetzung umfasst. Vorzugsweise beträgt der Anteil des wenigstens einen gefällten Silikats 12 bis 23 Gew.-%, noch mehr bevorzugt 14 bis 22 Gew.-%, weiter bevorzugt 16 bis 21 Gew.-%, jeweils bezogen auf die Gesamtzusammensetzung. Die Untergrenze des Anteilsbereichs beträgt höchst bevorzugt 17 oder 18 Gew.-%.

Weiterhin kann der Putzkörper der erfindungsgemäßen Zusammensetzung mindestens eine der folgenden Substanzen umfassen. Silikate, wie beispielsweise verschiedene Aluminiumsilikate und Zirkoniumsilikate, aber auch Natrium-Aluminiumsilikate, wie beispielsweise synthetische Zeolithe. Ebenso denkbar sind Silica Dimethyl Silylate oder Aluminiumoxid. Diese Substanzen können einzeln oder in beliebigen Mischungen davon vorliegen. Weitere, im Stand der Technik bekannte Putzkörpersubstanzen können zusätzlich in diesem bevorzugten Putzkörper umfasst sein.

In einer alternativen Ausführungsform der erfindungsgemäßen Zusammensetzung, enthält diese keine Aluminiumsilikate, Zirkoniumsilikate, Natrium-Aluminiumsilikate, wie beispielsweise synthetische Zeolithe, Silica Dimethyl Silylate und/oder Aluminiumoxid. Der Anteil an diesen - als Putzkörper fungierenden Verbindungen - beträgt, abgesehen von nicht vermeidbaren Verunreinigungen und im Bereich der üblichen Messgenauigkeit, somit 0 Gew.-%.

Die erfindungsgemäße Zusammensetzung weist ferner wenigstens eine oberflächenaktive Substanz auf. Der Begriff "oberflächenaktive Substanz" wird hier und im Folgenden synonym mit dem Begriff "Tensid" verwendet. Pharmazeutisch und/oder kosmetisch akzeptable Tenside sind dem Fachmann grundsätzlich bekannt. Es können beispielsweise anionische, kationische, nicht-ionische oder amphotere Tenside hierfür verwendet werden. Beispielshaft seien 1-Hexadecanol, 1-Octadecanol, Polyacrylsäure, Polyethylenglykol, Cocamidopropyl Betaine, Natriumlaurylsulfat, Natriumlaurylsarkosinat, Trinatriumphosphat, Natrium-methylcococyltaurat, Fettalkoholpolyglycoside, beispielsweise C₆-C₂₀-Alkylglykoside, einzeln oder in beliebigen Mischungen davon genannt.

In einer erfindungsgemäß bevorzugten Ausführungsform umfasst die Zusammensetzung als oberflächenaktive Substanz Natriumlaurylsulfat und/oder Natriumlaurylsarkosinat. In einer weiteren bevorzugten Ausführungsform umfasst die Zusammensetzung Cocamidopropyl Betaine als oberflächenaktive Substanz. Eine weitere bevorzugte Ausführungsform enthält eine Mischung aus Natriumlaurylsulfat und Cocamidopropyl Betaine als oberflächenaktive Substanzen. Das Gewichtsverhältnis von Natriumlaurylsulfat und Cocamidopropyl Betaine zueinander liegt vorzugsweise in dem Bereich von 4:1 (also 4 Teile Natriumlaurylsulfat und 1 Teil Cocamidopropyl Betaine) bis 1:4, noch mehr bevorzugt im Bereich von 3:1 bis 1:3, und höchst bevorzugt im Bereich von 2,5:1 bis 1:2,5.

Gemäß einer weiteren bevorzugten Ausführungsform umfasst die erfindungsgemäße Zusammensetzung als nichtionisches Tensid ein C₆-C₂₀-Alkylglykosid, vorzugsweise ein C₈-C₁₆-Alkylglykosid. Derartige Alkylglykoside sind im Handel beispielsweise unter dem Markennamen Plantacare erhältlich. Noch mehr bevorzugt liegt das C₆-C₂₀-Alkylglykosid, vorzugsweise das C₈-C₁₆-Alkylglykosid, in Kombination mit Natriumlaurylsulfat und/oder Natriumlaurylsarkosinat vor. In einer weiteren bevorzugten Ausführungsform liegt das C₆-C₂₀-Alkylglykosid, vorzugsweise das C₈-C₁₆-Alkylglykosid, in Kombination mit Cocamidopropyl Betaine. In einer weiteren bevorzugten Ausführungsform enthält die erfindungsgemäße Zusammensetzung eine Mischung aus Natriumlaurylsulfat, Cocamidopropyl Betaine und einem C₆-C₂₀-Alkylglykosid, vorzugsweise einem C₈-C₁₆-Alkyl-glykosid, als oberflächenaktive Substanzen. Das Gewichtsverhältnis von Natriumlaurylsulfat, Cocamidopropyl Betaine und dem C₆-C₂₀-Alkylglykosid, vorzugsweise dem C₈-C₁₆-Alkylglykosid, zueinander liegt vorzugsweise in dem Bereich von 0,5-1,5 Teilen Natriumlaurylsulfat, 0,1-1,0 Teilen Cocamidopropyl Betaine und 0,4-1,8 Teilen des C₆-C₂₀-Alkylglykosids, vorzugsweise des C₈-C₁₆-Alkylglykosids. Noch mehr bevorzugt liegt das Gewichtsverhältnis im Bereich von 0,8-1,2 Teilen Natriumlaurylsulfat, 0,2-0,8 Teilen Cocamidopropyl Betaine und 0,6-1,4 Teilen des C₆-C₂₀-Alkylglykosids, vorzugsweise des C₈-C₁₆- Alkylglykosids.

Den oberflächenaktiven Substanzen kommt in der erfindungsgemäßen Zusammensetzung sowohl eine Funktion bei der Bildung von Schaum als auch bei der Regelung der Viskosität der Zusammensetzung zu.

Der Anteil der wenigstens einen oberflächenaktiven Substanz in der erfindungsgemäßen Zusammensetzung beträgt 0,2 bis 4,0 Gew.-%, vorzugsweise 0,35 bis 3,0 Gew.-%, noch weiter bevorzugt 0,6 bis 2,5 Gew.-%, bezogen auf die Gesamtzusammensetzung. Alternativ kann der Anteil der wenigstens einen oberflächenaktiven Substanz in der erfindungsgemäßen Zusammensetzung 0,5 bis 3,0 Gew.-%, bezogen auf die Gesamtzusammensetzung, betragen.

Die erfindungsgemäßen Zusammensetzungen umfassen ferner wenigstens einen Viskositätsverstärker. Für Zahnreinigungs- und -pflegeprodukte grundsätzlich geeignete Viskositätsverstärker umfassen beispielhaft Cellulose, Cellulosederivate, Pflanzengummen, wie Xanthan Gum, Pectine, Carbomere, Polysaccharide aus Algen, beispielsweise Carrageenan, Polyvinylalkohol, Polyvinylpyrrolidon, Stärke und Stärkeether. Cellulosederivate sind beispielsweise Hydroxyalkylcellulosen, wie Hydroxyethylcellulose, Carboxymethylcellulose, Hydroxyethylcellulose, Methylhydroxypropylcellulose und deren Salze, beispielsweise Natriumsalze. Gemäß einer Ausführungsform ist der Viskositätsverstärker ausgewählt aus der Gruppe, bestehend aus Carboxymethylcellulose, Hydroxyethylcellulose, Xanthan Gum, Acrylate / C10-30-AlkylAcrylat-Crosspolymer und beliebige Mischungen davon.

Der Anteil des wenigstens einen Viskositätsverstärkers in der erfindungsgemäßen Zusammensetzung beträgt 0,05 bis 0,5 Gew.-%, bezogen auf die Gesamtzusammensetzung. Vorzugweise beträgt der Anteil des wenigstens einen Viskositätsverstärkers 0,1 bis 0,4 Gew.-%, noch mehr bevorzugt 0,1 bis 0,3 Gew.-%, jeweils bezogen auf die Gesamtzusammensetzung.

Die erfindungsgemäße Zusammensetzung umfasst weiterhin wenigstens ein Lösungsmittel. Das Lösungsmittel ist vorzugsweiseWasser. Alternativ kann das Lösungsmittel Wasser in einem Lösungsmittelgemisch umfassen. Vorzugsweise ist das Wasser für kosmetische oder pharmazeutische Formulierungen geeignet. Weitere pharmazeutisch und/oder kosmetisch akzeptable Lösungsmittel sind dem Fachmann bekannt.

Das Wasser ist mit einem Anteil von mindestens 10 Gew.-% in der Zusammensetzung umfasst und bildet den Rest auf 100 Gew.-% (ad. 100 Gew.-%), bezogen auf die Gesamtzusammensetzung. Das heißt, die Gew.-%-Anteile der wesentlichen und gegebenenfalls vorhandenen optionalen Bestandteile, einschließlich weitere optionaler Lösungsmittel außer Wasser, der Zusammensetzung sind immer so gewählt, dass sich in der Summe 100 Gew.-% ergeben.

Die Angabe "der Rest auf 100 Gew.-%" bzw. "ad. 100 Gew.-%" ist nicht so zu verstehen, dass dadurch in der erfindungsgemäßen Zusammensetzung optional nicht noch weitere Komponenten, beispielsweise Wirkstoffe und/oder Hilfs- oder Zusatzstoffe, als optionale Komponente(n) nicht mehr umfasst sein können. Das Vorliegen weiterer optionaler - auch nicht expliziter genannter - Bestandteile ist erfindungsgemäß ausdrücklich mit umfasst. Sollen derartige Bestandteile ausgeschlossen werden, wird hier und im Folgenden die Formulierung "bestehend aus" verwendet. Der "Rest" stellt immer den Anteil an Wasser dar, der nach Aufsummieren aller wesentlichen und, falls vorhanden, optionalen Bestandteilen notwendig ist, um dann 100 Gew.-% zu ergeben.

Der Anteil an Wasser in der erfindungsgemäßen Zusammensetzung beträgt vorzugsweise 10 bis 40 Gew.-%. Noch mehr bevorzugt liegt die Grenze des Wassergehalts bei 12 Gew.-% und/oder die Obergrenze bei 30 Gew.-%, mehr bevorzugt bei 25 Gew.-% und weiter bevorzugt bei 23 Gew.-%. Der Wassergehalt kann also beispielsweise erfindungsgemäß im Bereich von 12 bis 23 Gew.-% liegen.

Die erfindungsgemäße Zusammensetzung ist zur Reinigung und Pflege der Zähne, der Zahnhälse und des Zahnfleisches und insbesondere zur Vorbeugung und/oder Behandlung von Zahn- und Zahnfleischerkrankungen, insbesondere von Karies, Parodontose und Plaque geeignet. Im Besonderen ist die erfindungsgemäße Zusammensetzung zur Anwendung bei der Zahnreinigung und -pflege mittels einer Zahnputzvorrichtung für die gleichzeitige Reinigung mehrerer, vorzugsweise aller Zähne eines Benutzers, geeignet. Hierbei wird die erfindungsgemäße Zusammensetzung durch Aufbringen der Zusammensetzung auf einen Mundeinsatz oder mehrere Mundeinsätze der Zahnputzvorrichtung dargereicht.

Gemäß einer bevorzugten Ausführungsform umfasst die erfindungsgemäße Zusammensetzung als weiteres Feuchthaltemittel hydrogeniertes Stärkehydrolysat oder das weitere Feuchthaltemittel ist hydrogeniertes Stärkehydrolysat. Hydrogenierte Stärkehydrolysate (Hydrogenated Starch Hydrolysates) sind Mischungen mehrwertiger Alkohole wie beispielsweise Sorbitol, Maltitol und Zuckeralkohole höherer Ordnung. Ein erfindungsgemäß besonders bevorzugtes hydrogeniertes Stärkehydrolysat enthält zwischen 65 und 75 Gew-% Sorbitol. Entsprechende Produkte sind im Handel erhältlich. Der Anteil an hydrogeniertem Stärkehydrolysat an der Gesamtzusammensetzung ist in jedem Fall so zu wählen, dass die Menge an Glycerin und hydrogeniertem Stärke-hydrolysat den Anteil von 70 Gew.-% nicht übersteigt. Noch mehr bevorzugt liegt die Menge von Glycerin und hydrogeniertem Stärkehydrolysat im Bereich von 40 bis 70 Gew.-%, noch weiter bevorzugt im Bereich von 40 bis 60 Gew.-%. Es ist erfindungsgemäß auch vorgesehen, dass die Feuchthaltemittel-Komponente der erfindungs-gemäßen Zusammensetzung aus Glycerin und hydrogeniertem Stärkehydrolysat bestehen kann.

Unabhängig davon, ob die Feuchthaltemittel-Komponente aus Glycerin und hydrogeniertem Stärkehydrolysat besteht oder diese umfasst, liegt der Anteil an hydrogeniertem Stärkehydrolysat vorzugsweise im Bereich von 25 bis 40 Gew.-%, noch mehr bevorzugt im Bereich von 28 bis 38 Gew.-%. Alternativ kann das Gewichtsverhältnis von hydrogeniertem Stärkehydrolysat, insbesondere von hydrogeniertem Stärkehydrolysat mit einem Sorbitol-Anteil im Bereich von 65 bis 75 Gew.-%, zu Glycerin vorzugsweise in einem Bereich von 0,5:1 bis 5:1, noch mehr bevorzugt in einem Bereich von 0,8:1 bis 3:1, liegen.

Gemäß einer anderen bevorzugten Ausführungsform umfasst die erfindungsgemäße Zusammensetzung als weiteres Feuchthaltemittel Sorbitol oder das weitere Feuchthaltemittel ist Sorbitol. Es ist erfindungsgemäß also auch vorgesehen, dass die Feuchthaltemittel-Komponente der erfindungsgemäßen Zusammensetzung aus Glycerin und Sorbitol besteht.

Der Anteil an Sorbitol kann gemäß einer Ausführungsform vorzugsweise im Bereich von 16,25 bis 30 Gew.-%, noch mehr bevorzugt im Bereich von 20 bis 28 Gew.-%, noch mehr bevorzugt im Bereich von 20 bis 26 Gew.-% liegen. Alternativ kann der Anteil an Sorbitol an der erfindungsgemäßen Zusammensetzung 18,75 bis 26 Gew.-% betragen. Unabhängig davon, ob die Feuchthaltemittel-Komponente aus Glycerin und Sorbitol besteht oder diese beiden Bestandteile umfasst, liegt das Gewichtsverhältnis von Sorbitol zu Glycerin vorzugsweise in einem Bereich von 0,5:1 bis 5:1, noch mehr bevorzugt in einem Bereich von 0,8:1 bis 3:1.

In einer bevorzugten Ausführungsform ist die erfindungsgemäße Zusammensetzung somit frei von Xylit. Im Sinn der vorliegenden Erfindung bedeutet die Formulierung "frei von Xylit", dass die Zusammensetzung Xylit höchstens mit einem Anteil von weniger als 0,01 Gew.-%, bezogen auf die Gesamtzusammensetzung ohne Treibgas, aufweist. Der Anteil an Xylit liegt also im Bereich von 0 Gew.-% bis weniger als 0,01 Gew.-%, bezogen auf die Gesamtzusammensetzung ohne Treibmittel. Vorzugsweise beträgt die maximal zulässige Höchstgrenze 0,008 Gew.-%, noch mehr bevorzugt 0,006 Gew.-% und noch weiter bevorzugt 0,004 Gew.-%, jeweils bezogen auf die Gesamt-zusammensetzung ohne Treibmittel. Höchst bevorzugt ist der Anteil an Xylit in der Zusammensetzung 0 Gew.-%. Etwaige nicht vermeidbare Verunreinigungen sollen hierbei außer Acht bleiben. Ebenso gelten die üblichen Messgenauigkeiten.

Durch die Auswahl der vorstehend genannten Bestandteile in den genannten Mengen kann eine Zusammensetzung mit niedriger Viskosität bereitgestellt werden. Die niedrige Viskosität der Zusammensetzung ermöglicht eine gleichmäßige und sparsame Verteilung der Zusammensetzung auf allen Reinigungsflächen des Mundeinsatzes oder der Mundeinsätze. Es kommt dabei weder zu einer Überdosierung an Putzkörpern, was zu einer mechanischen Schädigung des Zahnschmelzes und zu einem unerwünschten Abrieb der Zähne führen könnte, noch schäumt die Zusammensetzung zu stark oder bildet einen zu stabilen, nicht mehr zerstörbaren Schaum aus.

Durch die Kombination der vorstehend genannten wesentlichen Bestandteile wird somit eine Zusammensetzung ermöglicht, die besonders zur gleichmäßigen, sparsamen und einfachen Applikation auf den Mundeinsatz oder die Mundeinsätze der Zahnputzvorrichtung geeignet ist. Durch die Kombination der vorstehend genannten wesentlichen Bestandteile kann die gewünschte Viskosität stabil eingestellt werden und darüber hinaus eine kurzzeitig stabile Schaumbildung bei der Applikation erreicht werden.

Gemäß einer bevorzugten Ausführungsform der erfindungsgemäßen Zusammensetzung weist diese ohne Treibgas eine dynamische Viskosität von 200 - 30.000 m Pa s auf. Vorzugsweise liegt die Viskosität der Zusammensetzung ohne Treibgas in einem Bereich von 400 - 10.000 m Pa s und noch mehr bevorzugt bei weniger als 10.000 m Pa s. So ist beispielsweise eine Viskosität im Bereich von 500 - 9.000 m Pa s bei 20°C, noch mehr bevorzugt. Noch weiter bevorzugt liegt die Viskosität in einem Bereich von 1.000 - 8.000 m Pa s bei 20°C. Der gewählte Viskositätsbereich stellt sicher, dass die erfindungsgemäße Zusammensetzung gleichmäßig und einfach auf den Mundeinsatz oder die Mundeinsätze der Zahnputzvorrichtung appliziert werden kann und auch genügend lange an diesem/diesen haftet.

Gemäß einer bevorzugten Ausführungsform enthält die erfindungsgemäße Zusammensetzung ein Gemisch aus mindestens 2, vorzugsweise 2 bis 4 der vorstehend im Detail beschriebenen gefällten Silikate. Noch mehr bevorzugt enthält das Gemisch drei gefällte Silikate. Das Gewichtsverhältnis der drei gefällten Silikate ist vorzugsweise im Bereich von 5-15 Teilen eines ersten Silikats, 2-8 Teilen eines zweiten Silikats und 0,5-5 Teilen eines dritten Silikats.

So können beispielsweise solche gefällten Silikate, die sich auch durch ihre verdickenden Eigenschaften auszeichnen, mit solchen gefällten Silikaten kombiniert werden, die als Putzkörper besonders geeignet sind.

Wie bereits vorstehend erläutert, unterscheiden sich die gefällten Silikate unter anderem in ihrer mittleren Teilchengröße und in ihrer Abrasivität (Härte). Im Rahmen der vorliegenden Offenbarung werden die gefällten Silikate deshalb in drei Kategorien 1 bis 3 eingeteilt.

Dabei sind gefällte Silikate der Kategorie 1 solche, die eine mittlere Teilchengröße in einem Bereich von 7 bis 8,5 µm und eine Einlehner Abrasion bzw. einen Messing-Einlehner-(BE)-Abrasionswert im Bereich von mehr als 5,0 bis 25 mg Verlust / 100 000 Umdrehungen, vorzugsweise im Bereich von 10-20 mg Verlust / 100 000 Umdrehungen ausweisen. Beispielhaft für ein gefälltes Silikat der Kategorie 1 sei ZEODENT^{®}103 genannt. Gefällte Silikate der Kategorie 2 sind als solche definiert, die eine mittlere Teilchengröße in einem Bereich von mehr als 8,5 bis 10 µm und eine Einlehner Abrasion im Bereich von mehr als 2,5 bis 5,0 mg Verlust / 100 000 Umdrehungen, vorzugsweise im Bereich von 2,7 bis 4,0 mg Verlust / 100 000 Umdrehungen ausweisen. Beispielhaft für ein gefälltes Silikat der Kategorie 2 sei ZEODENT^{®}115 genannt. Gefällte Silikate der Kategorie 3 sind als solche definiert, die eine mittlere Teilchengröße in einem Bereich von mehr als 10 bis 14 µm und eine Einlehner Abrasion im Bereich von weniger als 2,5 Verlust / 100 000 Umdrehungen, vorzugsweise im Bereich von 0,1 bis 2,3 mg Verlust / 100 000 Umdrehungen ausweisen. Beispielhaft für gefällte Silikate der Kategorie 3 seien ZEODENT^{®}165 und ZEODENT^{®}167 genannt.

Erfindungsgemäß besonders bevorzugt sind daher Gemische aus mindestens einem gefällten Silikat der Kategorie 1, beispielsweise ZEODENT^{®}103, mindestens einem gefällten Silikat der Kategorie 2, beispielsweise ZEODENT^{®}115, und mindestens einem gefällten Silikat der Kategorie 3, beispielsweise ZEODENT^{®}165 und/oder ZEODENT^{®} 167.

Ein Ziel des Einsatzes einer beliebigen Mischung von gefällten Silikaten und insbesondere von gefällten Silikaten aus den Kategorien 1 bis 3, ist es, einen mittleren bis hohen Abrasionsfaktor der Zahnpflege- und Zahnreinigungszusammensetzung und eine geeignete Verdickung der Zusammensetzung bereitzustellen. Der RDA-Wert der erfindungsgemäßen Zusammensetzungen liegt bevorzugt in einem Bereich von 30 bis 160, noch mehr bevorzugt in einem Bereich von 50 bis 130.

Da der Abrasionsfaktor und insbesondere der RDA-Wert einer Zahnreinigungszusammensetzung sowohl von der Abrasivität der verwendeten gefällten Silikate als auch von deren Anteil in der Zusammensetzung abhängt, sind die gefällten Silikate vorzugsweise mit einem Gewichtsverhältnis von 5-15 Teilen wenigstens eines gefällten Silikats der Kategorie 1, beispielsweise ZEODENT^{®}103, 2-8 Teilen wenigstens eines gefällten Silikats der Kategorie 2, beispielsweise ZEODENT^{®}115, und 0,5-5 Teilen wenigstens eines gefällten Silikats des Kategorie 3, beispielsweise ZEODENT^{®} 167, in dem Gemisch der gefällten Silikate enthalten. Noch mehr bevorzugt mit einem Gewichtsverhältnis von 7-13 Teilen wenigstens eines gefällten Silikats der Kategorie 1, beispielsweise ZEODENT^{®}103, 3-7 Teilen wenigstens eines gefällten Silikats der Kategorie 2, beispielsweise ZEODENT^{®}115, und wenigstens eines gefällten Silikats der Kategorie 3, beispielsweise 1,5-3,5 Teilen ZEODENT^{®}167. Ebenso ist es erfindungsgemäß bevorzugt, dass das wenigstens eine gefällte Silikat der Kategorie 1, beispielsweise ZEODENT^{®}103, mit einem Anteil von 6 bis 15 Gew.-%, vorzugsweise mit einem Anteil von 7 bis 13 Gew.-%, das wenigstens eine gefällten Silikats der Kategorie 2, beispielsweise ZEODENT^{®}115, mit einem Anteil von 3 bis 7 Gew.-%, und das wenigstens eine gefällte Silikat der Kategorie 3, beispielsweise ZEODENT^{®}167, mit einem Anteil von 1 bis 3,5 Gew.-%, vorzugsweise mit einem Anteil von 1,5 bis 3 Gew.-%, in der erfindungsgemäßen Zusammensetzung enthalten ist/sind.

Neben den vorstehend erwähnten gefällten Silikaten kann die erfindungsgemäße Zusammensetzung auch ein oder mehrere pyrogene Silikate umfassen. Pyrogene Silikate werden nicht nasschemisch, sondern durch ein thermisches Verfahren, vorzugsweise durch eine Flammenhydrolyse hergestellt und können ohne weitere Vermahlung oder Sprühtrocknung angewendet werden.

Die spezifische Oberfläche nach BET der pyrogenen Silikate liegt vorzugsweise im Bereich von 10 bis 1000 m²/g, noch mehr bevorzugt im Bereich von 20 bis 800 m²/g und höchst bevorzugt im Bereich von 40 bis 600 m²/g. Die mittlere Primärteilchengröße, also die Größe der kleinsten Bauteile, die im pyrogenen Silikat mehr oder minder stark aggregiert und agglomeriert sind, liegt vorzugsweise im Bereich von 2 bis 70 nm, noch mehr bevorzugt im Bereich von 5 bis 50 nm.

Die direkt nach dem thermischen Verfahren erhaltenen pyrogenen Silikate sind aufgrund der frei zugänglichen Silanolgruppen (Si-OH) auf der Partikeloberfläche hydrophil und lassen sich somit mit Wasser benetzen. In einer Ausführungsform der vorliegenden Erfindung ist das /sind die in der erfindungsgemäßen Zusammensetzung zusätzlich zu dem einen oder den mehreren gefällten Silikaten enthaltene(n) pyrogene(n) Silikat(e) hydrophil. Hydrophile pyrogene Silikate sind im Handel erhältlich und können beispielsweise unter dem Namen HDK^{®} N20 bezogen werden.

Erfindungsgemäß ist es aber in einer anderen Ausführungsform ebenfalls vorgesehen, dass das/die in der erfindungsgemäßen Zusammensetzung zusätzlich zu dem einen oder den mehreren gefällten Silikaten enthaltene(n) pyrogene(n) Silikat(e) oberflächenbehandelt ist/sind und damit hydrophobe Eigenschaften aufweist/aufweisen. Die Oberflächenbehandlung erfolgt vorzugsweise durch einen chemischen Nachbehandlungsschritt. Das so erzeugte hydrophobe Verhalten kommt durch die Reaktion von hydrophilen Silanolgruppen mit organischen Resten, vorzugsweise mit Silanen, beispielsweise Dichlordimethylsilan, Alkoxy-Silanen, Silazanen, Siloxanen, C₁-C₅-Kohlenstoffgruppen, beispielsweise CH₃-Gruppen, zustande. Hydrophobe pyrogene Silikate sind im Handel erhältlich und können beispielsweise unter dem Namen Aerosil R 812 S bezogen werden.

Gemäß einer weiteren erfindungsgemäßen Ausführungsform der vorliegenden Erfindung kann die Zusammensetzung auch eine Mischung von mindestens einem hydrophoben und mindestens einem hydrophilen pyrogenen Silikat enthalten.

Durch den Einsatz von mindestens einem pyrogenen Silikat kann die Zusammensetzung in ihrem thixotropen Verhalten vorteilhaft eingestellt werden. Thixotropie bezeichnet eine zeitabhängige Änderung der Fließeigenschaften von Fluiden, bei der die Viskosität infolge andauernder äußerer Einflüsse ab- oder zunimmt und erst nach beendigter Beanspruchung wieder in die Ausgangsviskosität zurückkehrt. Damit wird die Zusammensetzung mit der Dauer ihrer Deformation dünnflüssiger. Somit wird sichergestellt, dass bei geringer mechanischer Belastung, also beispielsweise während der Lagerung, die Viskosität der Zusammensetzung hoch genug ist, um ein Absetzen von Feststoffen, beispielsweise der Putzkörper, zu verhindern, während die Viskosität bei hoher mechanischer Belastung, also beispielsweise beim Austritt der Zusammensetzung aus einer Spendervorrichtung, gering sein soll, um einen einwandfrei aus der Spendervorrichtung austreten zu können. Sobald sich die Zusammensetzung auf dem Mundeinsatz befindet, soll sich aber wieder die höhere Viskosität einstellen, um ein Ablaufen zu verhindern. Darüber hinaus kann das bzw. können die pyrogene(n) Silikat(e) als Dispergierhilfe eingesetzt werden.

Das optional in Kombination mit dem oder den gefällten Silikat(en) in der Zusammensetzung umfasste pyrogene Silikat oder die ebenfalls optionale Mischung aus zwei oder mehr pyrogenen Silikaten ist in der Zusammensetzung vorzugsweise mit einem Anteil von 0,1 bis 2 Gew.-%, bezogen auf die Gesamtzusammensetzung, in der erfindungsgemäßen Zusammensetzung umfasst. Vorzugsweise beträgt der Anteil des wenigstens einen pyrogenen Silikats 0,2 bis 1,5 Gew.-%, noch mehr bevorzugt 0,2 bis 1,0 Gew.-%, jeweils bezogen auf die Gesamtzusammensetzung.

Gemäß einer weiteren bevorzugten Ausführungsform der erfindungsgemäßen Zusammensetzung enthält die Zusammensetzung kein Polyethylenglycol bzw. keine Polyethylenglycole oder der Anteil an Polyethylenglycol bzw. Polyethylenglycolen in der Zusammensetzung ist maximal 0,8 Gew.-%, vorzugsweise maximal 0,4 Gew.-%. Polyethylenglycole sind gemäß dieser erfindungsgemäßen Ausführungsform somit mit einem Anteil von 0 bis 0,8 Gew.-%, vorzugsweise mit einem Anteil von 0 bis 0,4 Gew.-% umfasst. Höchst bevorzugt ist der Anteil, abgesehen von nicht vermeidbaren möglichen Verunreinigungen und innerhalb der üblichen Messgenauigkeit, 0 Gew.-%.

Polyethylenglycole können je nach Molekulargewicht unterschiedliche Funktionen in der Zusammensetzung einnehmen. Polyethylenglycole, insbesondere solche mit Molekulargewichten von 1.500 bis 1.000.000 g/mol, können als Viskositätsverstärker fungieren, Polyethylenglycole mit kleinerem Molekulargewicht können grundsätzlich aber auch als Feuchthaltemittel eingesetzt werden. Die vorstehend genannten Anteile gelten unabhängig vom Molekulargewicht der Polyethylenglycole.

Gemäß einer weiteren bevorzugten Ausführungsform der erfindungsgemäßen Zusammensetzung umfasst diese als Viskositätsverstärker Xanthan Gum oder der Viskositätsverstärker ist Xanthan Gum. Xanthan Gum, auch als Xanthan-Gummi bezeichnet, ist ein hochmolekulares, von dem Mikroorganismus Xanthomonas campestris gebildetes, oder aus hydrolysierter Stärke erzeugtes Heteropolysaccharid. Im Wesentlichen besteht Xanthan-Gummi aus beta-1,4-verknüpften Glucose-Einheiten mit Seitenketten, die Mannose und Glucuronsäure enthalten. Über seine Eignung viskose Lösungen bilden zu können hinaus, hat sich Xanthan Gum in den erfindungsgemäßen Zusammensetzungen wegen seiner dispersionsstabilisierenden Wirkung und seiner thixotrophen Eigenschaften überraschenderweise als besonders vorteilhafter Viskositätsverstärker herausgestellt. Das eingesetzte Xanthan Gum kann natürlichen oder synthetischen Ursprungs sein.

Mit der Maßgabe, dass die Viskositätsverstärker-Komponente der erfindungsgemäßen Zusammensetzung Xanthan Gum umfasst, ist es besonders bevorzugt, wenn die Viskositätsverstärker-Komponente eine Mischung aus Xanthan Gum und Acrylaten und/oder Polyacrylaten, insbesondere vernetzte Polyacrylate, C10-30-Alkyl-Acrylat-Crosspolymer, umfasst oder aus dieser Mischung besteht. Es ist in beiden Fällen besonders bevorzugt, wenn das Gewichtsverhältnis von Xanthan Gum zu Acrylat(en) und/oder Polyacrylat(en), insbesondere vernetzte Polyacrylate, C10-30-Alkyl-Acrylat-Crosspolymer, in einem Bereich von 10:1 bis 1:10, noch mehr bevorzugt im Bereich von 8:1 bis 1:8, und höchst bevorzugt im Bereich von 6,5:1 bis 1:6,5 liegt.

Acrylate und/oder Polyacrylate, insbesondere vernetzte Polyacrylate, C10-30-AlkylAcrylat-Crosspolymer sind besonders geeignet für Tensidsysteme, die schwierig zu verdicken sind und sie stellen stabile Viskositäten über einen weiten pH-Bereich bereit. Darüber hinaus beeinflussen sie die Schaumeigenschaften positiv.

Aufgrund der vorteilhaften Eignung von Xanthan Gum und den Acrylaten und/oder Polyacrylaten, insbesondere von C10-30-Alkyl-Acrylat-Crosspolymeren, ist es daher erfindungsgemäß auch vorgesehen, dass die Zusammensetzung keine Cellulose und/oder Cellulosederivate und/oder Polysaccharide aus Algen, beispielsweise Carrageenan und/oder Alginat, enthält, also frei von einem oder mehrerer diese Bestandteile ist.

Die Cellulose kann, falls vorhanden, in nicht-derivatisierter Form vorliegen und wasserunlöslich sein. Eine Cellulose ist im Sinn der vorliegenden Erfindung dann wasserunlöslich, wenn in 100 g einer gesättigten wässerigen Lösung bei 20 °C weniger als 1 Gew.-% der Cellulose gelöst ist. Nicht-derivatisierte Cellulosen im Sinne der Erfindung sind solche, deren Hydroxylgruppen nicht durch eine andere chemische Gruppe substituiert sind. Cellulosederivate umfassen Hydroxyalkylcellulosen, wie Hydroxyethylcellulose, Carboxymethylcellulose, Hydroxyethylcellulose, Methylhydroxypropylcellulose und deren Salze, beispielsweise Natriumsalze. Carrageenan, auch als Carrageen bezeichnet, ist eine Sammelbezeichnung langkettiger Kohlenhydrate, die in Rotalgen vorkommen.

Im Sinn der vorliegenden Erfindung bedeutet die Formulierung "frei von Cellulose und/oder Cellulosederivate", dass die Zusammensetzung Cellulose, Cellulosederivate oder ein Gemisch von Cellulose und einem oder mehreren Cellulosederivaten höchstens mit einem Anteil von weniger als 0,01 Gew.-%, bezogen auf die Gesamtzusammensetzung ohne Treibgas, aufweist. Der Anteil an Cellulose, Cellulosederivate oder ein Gemisch von Cellulose und einem oder mehreren Cellulosederivaten liegt also im Bereich von 0 Gew.-% bis weniger als 0,01 Gew.-%, bezogen auf die Gesamtzusammensetzung ohne Treibmittel. Vorzugsweise beträgt die maximal zulässige Höchstgrenze 0,008 Gew.-%, noch mehr bevorzugt 0,006 Gew.-% und noch weiter bevorzugt 0,004 Gew.-%, jeweils bezogen auf die Gesamt-zusammensetzung ohne Treibmittel. Höchst bevorzugt ist der Anteil an Cellulose, Cellulosederivate oder einem Gemisch von Cellulose und einem oder mehreren Cellulosederivaten in der Zusammensetzung 0 Gew.-%. Etwaige nicht vermeidbare Verunreinigungen sollen hierbei außer Acht bleiben. Ebenso gelten die üblichen Messgenauigkeiten.

Entsprechendes gilt für die Formulierung "frei von Polysacchariden aus Algen, beispielsweise Carrageenan und/oder Alginat". Entsprechende Zusammensetzung enthalten Carrageenan und/oder Alginat und/oder andere Polysaccharide aus Algen, höchstens mit einem Anteil von weniger als 0,01 Gew.-%, bezogen auf die Gesamtzusammensetzung ohne Treibgas, aufweist. Der Anteil an Carrageenan und/oder Alginat und/oder anderer Polysaccharide aus Algen liegt also im Bereich von 0 Gew.-% bis weniger als 0,01 Gew.-%, bezogen auf die Gesamtzusammensetzung ohne Treibmittel. Vorzugsweise beträgt die maximal zulässige Höchstgrenze 0,008 Gew.-%, noch mehr bevorzugt 0,006 Gew.-% und noch weiter bevorzugt 0,004 Gew.-%, jeweils bezogen auf die Gesamtzusammensetzung ohne Treibmittel. Höchst bevorzugt ist der Anteil an Carrageenan und/oder Alginat und/oder anderer Polysaccharide aus Algen in der Zusammensetzung 0 Gew.-%. Etwaige nicht vermeidbare Verunreinigungen sollen hierbei außer Acht bleiben. Ebenso gelten die üblichen Messgenauigkeiten.

Weiterhin ist es erfindungsgemäß bevorzugt, dass die Zahnpflege- und Zahnreinigungszusammensetzung wenigstens einen Wirk- oder Inhaltsstoff umfasst. Der mindestens eine Wirkstoff ist beispielsweise ausgewählt aus der Gruppe, bestehend aus Hydroxylapatit oder weiteren remineralisierenden oder keramisierenden Stoffen, einem Fluoridwirkstoff, einem Mittel zur Zahnfleischpflege, einem desinfizierenden Bestandteil, einem Mittel zur Reduktion von Zahnbelägen, einem entzündungshemmenden Mittel, einem Konservierungsmittel und einer beliebigen Mischung davon.

Der Anteil des wenigstens einen oder der weiteren Wirkstoffe in der erfindungsgemäßen Zusammensetzung beträgt 0,5 bis 8 Gew.-%, bezogen auf die Gesamtzusammensetzung. Vorzugweise beträgt der Anteil des wenigstens einen oder der Wirkstoffe 1,5 oder 2,0 bis 7,5 Gew.-%, noch mehr bevorzugt 2,5 bis 7 Gew.-%, jeweils bezogen auf die Gesamtzusammensetzung.

Als Fluorid-Wirkstoff wird besonders bevorzugt ein Aminfluorid, Natriumfluorid oder Zinnfluorid oder eine Mischung davon verwendet. Der oder die Fluorid-Wirkstoffe sind vorzugsweise mit einem Anteil von 0,05 bis 1,5 Gew.-%, bezogen auf die Gesamtzusammensetzung enthalten. Noch mehr bevorzugt beträgt der Anteil 0,1 bis 1,2 Gew.-%, höchst bevorzugt liegt der Anteil im Bereich von 0,2 bis 1,0 Gew.-%.

Alternativ zum Fluorid-Wirkstoff kann die erfindungsgemäße Zusammensetzung Hydroxylapatit enthalten. In einer möglichen erfindungsgemäßen Ausführungsform enthält die Zahnpflegezusammensetzung entweder Fluoride, als weiteren Wirkstoff, oder Hydroxylapatit oder weitere remineralisierende Stoffe und Kombinationen aus Hydroxylapatit und einem oder mehreren weiteren remineralisierenden Stoffen.

Als Mittel zur Zahnfleischpflege wird besonders bevorzugt Allantoin, Panthenol, Extrakte aus Arnika, Myrrhe, Ratanhia, Salbei, Echinacea, Kamille, Rosmarinöl, Thymianöl oder Aloe oder eine beliebige Mischung von diesen Substanzen verwendet. Das Mittel zur Zahnfleischpflege ist, falls vorhanden, vorzugsweise mit einem Anteil von 0,1 bis 3 Gew.-%, noch mehr bevorzugt mit einem Anteil von 0,15 bis 2 Gew.-%, bezogen auf die Gesamtzusammensetzung enthalten.

Als Mittel zur Reduktion von Zahnbelägen kann beispielsweise Chlorhexidindigluconat, Zinkaspartat oder Arginin oder eine beliebige Mischung von diesen verwendet werden.

Das Mittel zur Reduktion von Zahnbelägen ist, falls vorhanden, vorzugsweise mit einem Anteil von 0,2 bis 3 Gew.-%, noch mehr bevorzugt mit einem Anteil von 0,5 bis 2 Gew.-%, bezogen auf die Gesamtzusammensetzung enthalten.

Weitere Wirkstoffe umfassen auch entzündungshemmende Mittel und desinfizierende Bestandteile, wie Bakterizide. Sie sind, falls vorhanden, vorzugsweise mit einem Anteil von 0,1 bis 3 Gew.-%, noch mehr bevorzugt mit einem Anteil von 0,2 bis 2 Gew.-%, bezogen auf die Gesamtzusammensetzung enthalten.

Es ist weiterhin erfindungsgemäß bevorzugt, dass die Zusammensetzung übliche Hilfs- und Zusatzstoffe ausgewählt aus der Gruppe, bestehend aus Aromastoffe, Süßungsmittel, Konservierungsmittel, Pigmente, Farbstoffe, Puffersubstanzen und einer beliebigen Mischung davon umfasst.

So kann die erfindungsgemäße Zusammensetzung beispielsweise ein oder mehrere Farbpigmente umfassen. Pharmazeutisch und/oder kosmetisch akzeptable Farbpigmente sind dem Fachmann bekannt. Diese können einzeln oder in beliebigen Mischungen davon eingesetzt werden. Vorzugsweise wird als Farbpigment Chlorophyllin (C.1. 75810), gegebenenfalls auch in Mischung mit weiteren Farbpigmenten eingesetzt. Der Anteil an Farbpigment, sei es ein Farbpigment oder eine Mischung aus mehreren Farbpigmenten, beträgt vorzugsweise 0 bis 1,0 Gew.-%, vorzugsweise 0,2 bis 1,0 Gew.-%, bezogen auf die Gesamtzusammensetzung. Noch mehr bevorzugt beträgt der Bereich 0,3 bis 0,8 Gew.-%.

Die erfindungsgemäße Zusammensetzung kann ferner einen oder mehrere Aromastoffe umfassen. Besonders bevorzugt umfasst der Aromastoff Anethol, Menthol, grüne Minze ("Spearmint"), Eucalypthus oder Limonen oder eine beliebige Kombination von diesen bevorzugten Aromastoffen. Der Anteil an Aromastoffen, sei es ein Aromastoff oder eine Mischung aus mehreren Aromastoffen, beträgt vorzugsweise 0 bis 1,5 Gew.-%, vorzugsweise 0,1 bis 1,3 Gew.-%, bezogen auf die Gesamtzusammensetzung. Noch mehr bevorzugt beträgt der Anteil 0,2 bis 1,1 Gew.-%.

Als weitere optionale Bestandteile kann die erfindungsgemäße Zusammensetzung umfassen wenigstens ein Bindemittel, wenigstens einen Lösungsvermittler, wenigstens ein Netzmittel, wenigstens einen Süßstoff, wenigstens ein Bleichmittel und/oder wenigstens einen pH-Regler. Die erfindungsgemäße Zusammensetzung kann als optionalen Bestandteil auch einen oder mehrere weitere Stabilisatoren, wie beispielsweise Natriumgluconat (INCI: Sodium Gluconate) umfassen. Dieser wenigstens eine weitere Stabilisator kann eine Entmischung der Emulsion verhindern und/oder eine Zersetzung der anderen Bestandteile der Zusammen-setzung, beispielsweise der Wirkstoffe, verhindern. Natriumgluconat verhindert beispielsweise nicht nur eine Entmischung der erfindungsgemäßen Zusammensetzung, es verhindert darüber hinaus auch eine oxidative Zersetzung der weiteren Bestandteile, insbesondere der Wirkstoffe, der Zusammensetzung. Diese optionalen Bestandteile können einzeln oder in beliebigen Kombinationen miteinander in der erfindungsgemäßen Zusammensetzung umfasst sein.

Die Wirkstoffe und Hilfs- oder Zusatzstoffe der Zusammensetzung zur Mund- und/oder Zahnreinigung und -pflege können grundsätzlich die sein, wie sich auch für bereits bekannte Zahnreinigungsmittel, wie etwa Zahncremes, Zahnreinigungsgelen und dergleichen, eingesetzt werden. Dennoch haben sich bestimmte Inhalts- und Hilfs- oder Zusatzstoffe und/oder deren Kombination miteinander als besonders vorteilhaft herausgestellt, um eine Zusammensetzung bereitzustellen, die gleichmäßig, sparsam und einfach auf den Mundeinsatz oder die Mundeinsätze der Zahnputzvorrichtung zum gleichzeitigen Reinigen von mehrerer, vorzugsweise aller Zähne eines Benutzers, appliziert werden kann. Darüber hinaus stellt die Zusammensetzung sicher, dass genügend Wirkstoff appliziert wird, aber gleichzeitig eine Überdosierung vermieden wird.

Alle Bestandteile der Zusammensetzung sind in einem Reinheitsgrad, der für Zahnpflegezusammensetzungen geeignet und notwendig ist. Werden für bestimmte Hilfs- oder Zusatzstoffe hierin keine Angaben zu deren Gew.-%-Anteil gemacht, bedeutet dies, dass der Fachmann die bevorzugten Bereiche kennt oder einfach ermitteln kann.

Der pH-Wert der erfindungsgemäßen Zahnpflege- und Zahnreinigungszusammensetzung liegt vorzugsweise im Bereich von 5,5 bis 9,0, noch mehr bevorzugt im Bereich von 6,0 bis 8,0 und am bevorzugtesten im Bereich von 6,5 bis 7,5.

Ebenfalls im Umfang der vorliegenden Erfindung mit umfasst sind solche Zusammensetzungen, die, mit Ausnahme von üblichen Verunreinigungen, nur aus den vorstehend genannten wesentlichen, und gegebenenfalls einem oder mehrerer der optionalen Bestandteile, bestehen.

Die Angabe Gew.-%, wie in der vorliegenden Patentanmeldung verwendet, bedeutet, sofern nichts anderes angegeben wird, dass der Anteil auf das Gesamtgewicht der Zusammensetzung ohne Treibmittel bezogen ist.

Eine besonders bevorzugte erfindungsgemäße Zusammensetzung umfasst:
- 20 bis 50 Gew.-%, vorzugsweise 25 bis 40 Gew.-%, hydrogeniertes Stärkehydrolysat, das vorzugsweise einen Sorbitol-Anteil von 65 bis 75 Gew.-% aufweist,
- 15 bis 30 Gew.-%, Glycerin,
- 1 bis 2 Gew.-% aminfluoridhaltiger Rohstoff mit 20-50% Wirkstoffanteil,
- 2 bis 20 Gew.-%, vorzugsweise 6 bis 15 Gew.%, gefälltes Silikat der Kategorie 1, vorzugsweise Zeodent 103,
- 1 bis 10 Gew.-%, vorzugsweise 3 bis 7 Gew.-%, gefälltes Silikat der Kategorie 2, vorzugsweise Zeodent 115,
- 1 bis 10 Gew.-%, vorzugsweise 1 bis 3 Gew.-%, gefälltes Silikat der Kategorie 3, vorzugsweise Zeodent 167,
- 0,1 bis 2 Gew.-%, vorzugsweise 0,2 bis 1 Gew.-%, Natriumlaurylsulfat,
- 0,1 bis 2 Gew.-%, vorzugsweise 0,15 bis 1 Gew.-%, Cocamidopropylbetain,
- 0,05 bis 0,2 Gew.-% Xanthan Gum,
- sowie mindestens 10 Gew.-% Wasser ad 100 Gew.-%.

Eine weitere besonders bevorzugte erfindungsgemäße Zusammensetzung umfasst:
- 16,25 bis 30 Gew.-% Sorbitol,
- 15 bis 30 Gew.-% Glycerin,
- 1 bis 2 Gew.-% aminfluoridhaltiger Rohstoff mit 20-50% Wirkstoffanteil,
- 6 bis 13 Gew.%, vorzugsweise 7 bis 13 Gew.-%, gefälltes Silikat der Kategorie 1, vorzugsweise Zeodent 103,
- 3 bis 7 Gew.-% gefälltes Silikat der Kategorie 2, vorzugsweise Zeodent 115,
- 1 bis 3,5 Gew.-%, vorzugsweise 1,5 bis 3 Gew.-%, gefälltes Silikat der Kategorie 3, vorzugsweise Zeodent 167,
- 0,1 bis 2 Gew.-%, vorzugsweise 0,2 bis 1 Gew.-%, Natriumlaurylsulfat,
- 0,1 bis 2 Gew.-%, vorzugsweise 0,15 bis 1 Gew.-%, Cocamidopropylbetain,
- 0,05 bis 0,2 Gew.-% Xanthan Gum,
- sowie mindestens 10 Gew.-% Wasser.

In diesen beiden besonders bevorzugten erfindungsgemäßen Zusammensetzung werden die Anteile der gefällten Silikate, des Glycerins, der oberflächenaktiven Substanzen, der Viskositätsverstärker jeweils so gewählt, dass die vorstehend offenbarten Gew.-%-Anteile von 10 bis 23,5 Gew.-% gefälltes Silikat, 15 bis 30 Gew.-% Glycerin, 0,2 bis 4,0 Gew.-% oberflächenaktive Substanz und 0,05 bis 0,5 Gew.-% Viskositätsverstärker und mindestens 10 Gew.-% Wasser eingehalten werden. In einer Ausführungsform der vorliegenden Erfindung besteht die Basisformulierung der Zusammensetzung aus den im vorgehenden Abschnitt genannten Komponenten mit den angegebenen Anteilen. Dieser Basisformulierung können zur Bereitstellung der fertigen Zusammensetzung ohne Treibgas ergänzend zu diesen Komponenten wenigstens ein Wirkstoff, beispielsweise ausgewählt aus der Gruppe, bestehend aus Hydroxylapatit oder weiteren remineralisierenden oder keramisierenden Stoffen, einem Fluoridwirkstoff, einem Mittel zur Zahnfleischpflege, einem desinfizierenden Bestandteil, einem Mittel zur Reduktion von Zahnbelägen, einem entzündungshemmenden Mittel, einem Konservierungsmittel und einer beliebigen Mischung davon, und/oder wenigstens ein üblicher Hilfs- und Zusatzstoffe ausgewählt aus der Gruppe, bestehend aus Aromastoffe, Süßungsmittel, Konservierungsmittel, Pigmente, Farbstoffe, Puffersubstanzen und einer beliebigen Mischung davon, in den vorstehend spezifizierten Anteilen, sowie ein Mittel zum Einstellen des pH-Wertes zugesetzt werden.

Diese besonders bevorzugte Zusammensetzung kann entweder, abgesehen von üblichen Verunreinigungen, nur aus diesen Bestandteilen bestehen, oder sie kann darüber hinaus noch 0,3 bis 1,0 Gew.-% Chlorophyllin und/oder 0,5 bis 1,5 Gew% Anethol oder Menthol oder Limonen oder eine beliebige Mischung davon umfassen. Darüber hinaus können in der Zusammensetzung noch umfasst sein Saccharin oder ein anderer Süßstoff, ein pH-Regler, PEG-40 hydriertes Rizinusöl (INCI: PEG-40 Hydrogenated Castor Oil) und/oder Natriumgluconat (INCI: Sodium Gluconate). Gemäß einer weiteren erfindungsgemäßen Ausführungsform besteht die Zusammensetzung, abgesehen von üblichen Verunreinigungen, nur aus den genannten wesentlichen und einem oder mehrerer der genannten optionalen Bestandteile.

Bei der Verwendung der erfindungsgemäßen Zusammensetzung ist es erfindungsgemäß vorzugsweise vorgesehen, dass die erfindungsgemäße Zusammensetzung ausgehend von der aufschäumbaren Mischung in einem zweiten Stadium in einen Schaum, vorzugsweise in ein Schaumspray, mit einer bestimmten Lebensdauer oder in ein Aerosol überführt wird. Deshalb kommt den oberflächenaktiven Substanzen und/oder den Viskositätsverstärkern in der Zusammensetzung eine besondere Bedeutung zu. Bezüglich besonders bevorzugter Substanzen und Kombinationen davon wird auf die vorstehende Offenbarung verwiesen, in der diese Substanzen im Einzelnen diskutiert werden.

Gemäß einem weiteren Aspekt betrifft die vorliegende Erfindung also die vorstehend im Detail beschriebene Zahnpflege- und Zahnreinigungszusammensetzung in Kombination mit einem in der Zusammensetzung enthaltenen Treibgas oder einem Treibgasgemisch. Vorzugsweise ist das Treibgas oder Treibgasgemisch in der erfindungsgemäßen Zusammensetzung in einer Menge von wenigstens 0,2 Gew.-%, noch mehr bevorzugt von wenigstens 0,5 Gew.-%, noch mehr bevorzugt von wenigstens 1 Gew.-% enthalten. Weiterhin ist es bevorzugt, dass der Gehalt an Treibgas oder Treibgasgemisch in der Zusammensetzung nicht höher als 15 Gew.-% und noch mehr bevorzugt nicht höher als 13 Gew.-% ist, da das Mittel ansonsten bei der Applikation und Verwendung einen zu voluminösen Schaum bilden würde. Das Treibgas oder Treibgasgemisch fungiert also als Schaumbildner. Am bevorzugtesten ist es, dass der Gehalt an Treibmittel oder Treibmittelgemisch in der Zusammensetzung in einem Bereich von 2,5 bis 12 Gew.-% liegt.

Die vorliegende Erfindung betrifft somit in einem weiteren Aspekt auch ein Aerosol oder einen Schaum, das/der aus der erfindungsgemäßen Zusammensetzung gebildet wird und das/der gleichmäßig auf den Mundeinsatz oder die Mundeinsätze verteilt werden kann. Das Aerosol oder der gebildete Schaum ist für einen ausreichenden Zeitraum stabil und haftet damit für einen ausreichenden Zeitraum an den Mundeinsätzen. Ein Abfließen der Zusammensetzung bzw. des daraus gebildeten Schaums oder der Schaumtropfen oder der Aerosol-tropfen vor dem Einsetzen des Mundeinsatzes oder der Mundeinsätze in den Mund kann somit vermieden werden.

Aerosole und Schäume bieten den Vorteil einer langen Haltbarkeit, einer exakten Dosierung und einer sparsamen Anwendung. Darüber hinaus können in den Spender keine Mikroorganismen eindringen, so dass auch nicht konservierte Produkte möglich sind.

Ein Aerosol ist ein heterogenes Gemisch nicht gasförmiger Stoffe in einem Gas. Es entsteht erst durch Betätigung des Auslassventils einer geeigneten Spender- und Speichervorrichtung für die erfindungsgemäße Zahnreinigungs- und -pflegezusammensetzung und wird damit erst bei Bedarf und in der benötigten Menge hergestellt. Der Schaum ist das finale Produkt, welches durch die Spender- und Speichervorrichtung aus der erfindungsgemäßen Zusammensetzung gebildet wird. Ein Schaum umfasst gasförmige Bläschen, die von festen oder flüssigen Wänden eingeschlossen sind. Die in der Spendervorrichtung enthaltene Zusammensetzung sollte Tenside oder ähnliche Vernetzungsmittel, sowie vorzugsweise Schaumstabilisatoren enthalten, die einen ausreichend stabile Schaumerzeugung ermöglichen. In Abhängigkeit der speziellen Eigenschaften der in der Zusammensetzung enthaltenen oberflächenaktiven Stoffe entstehen Schaumbläschen mit unterschiedlicher Größe, Wandstärke und Lebensdauer. Grundsätzlich ist die Lebensdauer von flüssigen Schäumen begrenzt. Diese kann von der Viskosität der den Schaum bildenden Flüssigkeit, also der hierin beschriebenen Zusammensetzung, und/oder den in der Zusammensetzung enthaltenen Stoffen abhängen.

Das aus dem Spender austretende Aerosol oder der aus dem Spender austretende Schaum expandiert dann weiter und kann somit die für die Zähne vorgesehenen Aussparungen, hierin auch als U-förmige Wannen beschrieben, in den Mundeinsätzen mehr oder weniger vollständig ausfüllen. Damit wird die Zusammensetzung gleichmäßig verteilt, womit sichergestellt ist, dass die Aktivstoffe gleichmäßig verteilt sind und damit alle Zähne effizient gereinigt und behandelt werden können. Gleichzeitig bietet das Aerosol oder der Schaum den Vorteil, dass die Zusammensetzung sparsam appliziert und eine Überdosierung vermieden werden kann.

Gemäß einem weiteren Aspekt betrifft die vorliegende Erfindung ein System, welches die hierin beschriebene erfindungsgemäße Zahnpflege- und Zahnreinigungszusammensetzung, und einen Behälter bzw. Spender mit Mitteln zur Bevorratung und Abgabe der Zahnpflege- und Zahnreinigungszusammensetzung umfasst. Die Zahnpflege- und Zahnreinigungszusammensetzung kann mit einem Treibgas oder Treibgasgemisch versetzt sein.

Damit solche Systeme ein geeignetes Aerosol bzw. einen geeigneten Schaum produzieren und abgeben können, spielen neben der Zusammensetzung noch andere technische Parameter wie beispielsweise das Verhältnis von Wirkstofflösung zu Treibgas, die Art des Treibgases, die Größe der Ventilöffnung und/oder die Größe der Sprühkopföffnung eine Rolle. Die Größe der Ventilöffnung und die Größe der Sprühkopföffnung stehen in direkter Korrelation zur Tröpfchengröße des Aerosols oder des Schaums. Für den Ventilkopf liegt sie vorzugsweise zwischen 0,2 mm und 4 mm, für den Sprühkopf vorzugsweise zwischen 0,4 mm und 6 mm.

Erfindungsgemäß umfasst der Spender ein Mittel zur Bevorratung der erfindungsgemäßen Zusammensetzung, ein Fördersystem zum Befördern der Zusammensetzung und ein Auslassventil zum Abgeben der Zusammensetzung. Das Fördersystem befördert die Zusammensetzung vorzugsweise mittels eines Treibgases, Druckluft oder über ein Pumpsystem nach außen. Der Spender ist dabei ausreichend druckstabil gegenüber beispielsweise dem Expansionsdruck der treibgashaltigen Zusammensetzung oder des Treibgases ausgebildet. Die Zusammensetzung und der Spender wirken derart zusammen, dass bei Betätigung des Auslassventils das treibgashaltige Zahnreinigungsmittel in strangförmiger, wenig aufgeschäumter Form aus dem Auslassventil austritt und zeitlich verzögert am Applikationsort weiter aufschäumt bzw. expandiert.

In einer erfindungsgemäßen Ausführungsform befördert das Fördersystem die Zusammensetzung mittels eines Treibgases oder Treibgasgemisches, Druckluft oder über ein Pumpsystem aus dem Spender. Die Austrittsöffnung des Spenders, beispielsweise die Schaumdüse, ist vorzugsweise derart ausgestaltet, dass eine gleichmäßige, zielgenaue und sparsame Verteilung der erfindungsgemäßen Zusammensetzung auf dem Mundeinsatz oder den Mundeinsätzen gewährleistet wird. Das Auslassventil ist vorzugsweise manuell betätigbar.

Gemäß einer bevorzugten Ausführungsform ist der Spender eine Aerosoldose bzw. Spraydose. Hierbei handelt es sich vorzugsweise um einen metallischen Behälter oder vorzugsweise um einen Kunststoffbehälter, der die erfindungsgemäße Zusammensetzung enthält und aufgrund des Einsatzes eines Treibgases oder Treibgasgemisches unter Druck steht. Durch den Innendruck der Spraydose wird der Inhalt genau dann freigesetzt, wenn man das Auslassventil betätigt. Ein Teil des Treibgases oder Treibgasgemisches liegt gelöst in der Wirkstofflösung, also der eigentlichen Zusammensetzung der vorliegenden Erfindung, vor und der andere Teil des Treibgases oder Treibgasgemisches liegt als Gas vor. Wird das Auslassventil nun betätigt, treibt der gasförmige Anteil des Treibgases oder Treibgasgemisches, die Wirkstofflösung durch das Ventil nach außen. Jetzt verdampft das in der Zusammensetzung enthaltene Treibgas oder Treibgasgemisch extrem schnell und die zurückbleibende Wirkstofflösung verteilt sich unter Ausbildung eines Schaums sehr fein und gleichmäßig. Als Treibgas für eine Aerosoldose kann vorzugsweise Luft, Kohlendioxid, Distickstoffoxid (N₂O), n-Propan, n-Butan, n-Pentan, Isopentan, Isopropan, Isobutan oder eine beliebige Mischung von diesen eingesetzt werden. Die Menge an Treibgas oder Treibgasgemisch im Spender ist so gewählt, dass sichergestellt werden kann, dass die Dose vollständig entleert werden kann und bis zum Entleeren der Dose immer genügend Treibgas oder Treibgasgemisch in flüssiger Form in der Zusammensetzung vorliegt und als Schaumbildner fungieren kann.

Gemäß einer alternativen bevorzugten Ausführungsform ist der Spender eine Bag-on-Valve Dose. Mit dieser Aerosoltechnologie lassen sich auch dickflüssigere Produkte austragen. Hierbei sind Treibgas oder Treibgasgemisch und Wirkstofflösung, also die vorstehend beschriebene erfindungsgemäße Zahnpflege- und Zahnreinigungszusammensetzung, voneinander getrennt und liegen in zwei getrennten Kammern vor. Ein Beutel, beispielsweise ein Aluminiumbeutel oder ein laminierter Aluminiumbeutel, beinhaltet die Wirkstofflösung und dieser Beutel ist mit dem Ventil verbunden. Ein Treibgas oder Treibgasgemisch, beispielsweise Druckluft, Stickstoff, Kohlendioxid, Distickstoffoxid (N₂O), n-Propan, n-Butan, n-Pentan, Isopropan, Isobutan, Isopentan, ein Propan-Butan-Gemisch oder ein Butan-Pentan-Gemisch, umgibt den Beutel und liefert den notwendigen Druck zum Herausbefördern der Zusammensetzung aus dem Spender. Bei der Betätigung des Auslassventils quetscht das Treibgas nun Produkt aus dem Beutel. Die äußere Dose besteht vorzugsweise aus Weißblech, Aluminium oder Polyethylen-terephthalat, weil diese Materialien stabil, leicht und sehr gut recycelbar sind. Es ist erfindungsgemäß bevorzugt, dass die Bag-on-Valve Dose zur Bevorratung der erfindungs-gemäßen Zahnpflege- und Zahnreinigungszusammensetzung einen einzigen Beutel mit der fertigen Zusammensetzung enthält und nicht Teilmischungen in getrennten Beuteln bevorratet werden und erst bei Betätigung des Abgabeventils miteinander gemischt werden.

Gemäß einer besonders bevorzugten Ausführungsform einer Bag-on-Valve Dose bzw. eines Bag-on-Valve Systems befindet sich auch in der erfindungsgemäßen Zusammensetzung ein kleiner Anteil an Treibgas oder eines Treibgasgemisches. Dieser Anteil liegt vorzugsweise in dem Bereich von 0 bis 8 Gew.-%, noch mehr bevorzugt in einem Bereich von 0,5 bis 7 Gew.-% und am bevorzugtesten in einem Bereich von 1,5 bis 6 Gew.-%.

Das Treibgas ist vorzugsweise ausgewählt aus der Gruppe, bestehend aus Isobutan, n-Butan, Isopentan, n-Pentan, Lachgas (N₂O) oder einer beliebigen Mischung davon.

In einer erfindungsgemäßen Ausführungsform ist das Treibgas Distickstoffoxid (Lachgas). Das Distickstoffoxid wird in einer Menge eingesetzt, dass der resultierende Druck in der Spendervorrichtung vorzugsweise mindestens 5 bar beträgt. Noch mehr bevorzugt liegt der Druck in der Spendervorrichtung im Bereich von 6 bis 10 bar.

Distickstoffoxid wird aufgrund seines sehr niedrigen Siedepunkts im Sinne der vorliegenden Offenbarung als eine nicht komprimierbare Flüssigkeit verstanden. Im Gegensatz dazu sind beispielsweise n-Butan, Isobutan, n-Pentan und Isopentan komprimierbare Flüssigkeiten.

In einer weiteren erfindungsgemäßen Ausführungsform ist das Treibgasgemisch ein Gemisch aus n-Butan, Isobutan, n-Pentan und Isopentan. Vorzugsweise weist das Gemisch einen Anteil von Isopentan von mindestens 70 % und einen Anteil von Isobutan von mindestens 20 % auf. n-Pentan und n-Butan sind jeweils höchstens mit einem Anteil von 5 %, noch mehr bevorzugt von höchstens 3,5 % enthalten. Noch mehr bevorzugt umfasst das Treibgasgemisch 0,1-2 % n-Butan, 22-28 % Isobutan, 0,1-3 % n-Pentan und 72-78 % Isopentan auf. Die Anteile der vier Komponenten sind in dem Gemisch jeweils so gewählt, dass in Summe 100 % für das Treibgasgemisch erreicht werden. Die Einsatzkonzentration eines solchen Gemisches liegt vorzugsweise im Bereich von 2 bis 12 Gew.-%, noch mehr bevorzugt im Bereich von 3 bis 10 Gew.-%.

Das Treibgas oder das Treibgasgemisch ist gemäß einer weiteren, bevorzugten Ausführungsform der vorliegenden Erfindung so ausgewählt oder zusammengesetzt, dass es bei Normaldruck (1,013 hPa) einen Siedepunkt von -10 °C oder höher, noch mehr bevorzugt von -8 °C oder höher aufweist. Gemäß einer Ausführungsform der Erfindung ist das Treibgas n-Butan. Vorzugsweise liegt die Konzentration von n-Butan, bezogen auf die Zusammensetzung ohne Treibgas, also die reine Flüssigformulierung, im Bereich von 2 bis 12 Gew.-%, noch mehr bevorzugt im Bereich von 3 bis 10 Gew.-%.

Das in der erfindungsgemäßen Zusammensetzung enthaltene Treibgas oder Treibgasgemisch fungiert als Schaumbildern.

Auch für die Ausführungsform der Aerosoldose werden für die Herstellung eines Schaums oder Aerosols als Treibgas vorzugsweise n-Propan, n-Butan, n-Pentan, Isopropan, Isobutan, Isopentan oder beliebige Mischungen davon verwandt. Alternativ können auch Luft, Kohlendioxid, Distickstoffoxid oder beliebige Mischungen davon als Treibmittel eingesetzt werden. Es ist wiederum besonders bevorzugt, dass das Treibmittel oder das Treibmittelgemisch so ausgewählt ist, dass es keine Verbindung enthält, die bei Normaldruck einen Siedepunkt von weniger als -10 °C, noch mehr bevorzugt von weniger als -8 °C aufweist.

Auch für die Ausführungsform der Aerosoldose ist das Treibgas in einer erfindungsgemäßen Ausführungsform Distickstoffoxid (Lachgas). Das Distickstoffoxid wird in einer Menge eingesetzt, dass der resultierende Druck in der Spendervorrichtung vorzugsweise mindestens 5 bar beträgt. Noch mehr bevorzugt liegt der Druck in der Spendervorrichtung im Bereich von 6 bis 10 bar.

In einer weiteren erfindungsgemäßen Ausführungsform ist das Treibgasgemisch in der Aerosoldose ein Gemisch aus n-Butan, Isobutan, n-Pentan und Isopentan. Vorzugsweise weist das Gemisch einen Anteil von Isopentan von mindestens 70 % und einen Anteil von Isobutan von mindestens 20 % auf. n-Pentan und n-Butan sind jeweils höchstens mit einem Anteil von 5 %, noch mehr bevorzugt von höchstens 3,5 % enthalten. Noch mehr bevorzugt umfasst das Treibgasgemisch 0,1-2 % n-Butan, 22-28 % Isobutan, 0,1-3 % n-Pentan und 72-78 % Isopentan auf. Die Anteile der vier Komponenten sind in dem Gemisch jeweils so gewählt, dass in Summe 100 % für das Treibgasgemisch erreicht werden. Die Einsatzkonzentration eines solchen Gemisches liegt vorzugsweise im Bereich von 2 bis 12 Gew.-%, noch mehr bevorzugt im Bereich von 3 bis 10 Gew.-%, bezogen auf die Zusammensetzung ohne Treibgas, also die reine Flüssigformulierung.

Das Treibgas oder das Treibgasgemisch der Aerosoldose ist gemäß einer weiteren, bevorzugten Ausführungsform der vorliegenden Erfindung so ausgewählt oder zusammengesetzt, dass es bei Normaldruck (1,013 hPa) einen Siedepunkt von -10 °C oder höher, noch mehr bevorzugt von -8 °C oder höher aufweist. Gemäß einer Ausführungsform der Erfindung ist das Treibgas n-Butan. Vorzugsweise liegt die Konzentration von n-Butan, bezogen auf die Zusammensetzung ohne Treibgas, also die reine Flüssigformulierung, im Bereich von 2 bis 12 Gew.-%, noch mehr bevorzugt im Bereich von 3 bis 10 Gew.-%.

Eine Alternative zur Herstellung eines Schaums oder eines Aerosols aus der Zusammensetzung stellt die Verwendung eines manuellen oder elektrischen Pumpsystems dar. Wird die Pumpe betätigt, entsteht ein Überdruck zwischen einem äußerem und einem inneren Behälter, der nach Betätigung des Ventils das Aerosol bzw. den Schaum bildet. Die Anwendung der Zusammensetzung als Schaum ist besonders bevorzugt.

In einem weiteren Aspekt betrifft die vorliegende Erfindung die Verwendung des vorstehend beschriebenen Systems zur Bevorratung und Abgabe der erfindungsgemäßen Zahnpflege- und Zahnreinigungszusammensetzung. Dabei wird die Zusammensetzung vorzugsweise als zunächst nur wenig aufgeschäumter Strang auf den Mundeinsatz oder die Mundeinsätze der Zahnreinigungsvorrichtung aufgebracht. Nach der Applikation weist der Strang bei 20 °C und Normaldruck vorzugsweise eine Expansionsrate von mehr als 100 Vol.-% in 3 bis 5 Sekunden auf. Noch mehr bevorzugt liegt die Expansionsrate bei 20 °C und Normaldruck nach 3 bis 5 Sekunden in einem Bereich von 150 Vol.-% bis 1000 Vol.-%. Der so erhaltene Schaum wird dann zur Reinigung von Zahnoberflächen, Zahnzwischenräumen und der Mundhöhle verwendet.

In einem noch weiteren Aspekt betrifft die vorliegende Erfindung ein Verfahren zum gleichzeitigen Reinigung mehrerer, vorzugsweise aller Zähne mittels einer Vibrationszahnputzvorrichtung für die gleichzeitige Reinigung mehrerer, vorzugsweise aller Zähne eines Benutzers, wobei die Zusammensetzung dargereicht wird durch Aufbringen der Zusammensetzung auf einen Mundeinsatz oder mehrere Mundeinsätze der Zahnputzvorrichtung. Die Zahnreinigung dient insbesondere zum Vorbeugen und/oder Behandeln von Erkrankungen der Zähne, der Zahnhälse und/oder des Zahnfleisches.

Das Verfahren gemäß der vorliegenden Offenbarung umfasst entsprechend die folgenden Schritte:
- Bereitstellen einer Zahnpflege- und Zahnreinigungszusammensetzung, die als wesentliche Komponenten umfasst: 10 bis 40 Gew.-%, vorzugsweise 15 bis 30 Gew.-% Glycerin, 10 bis 23,5 Gew.-% wenigstens eines gefällten Silikats, vorzugsweise 10 bis 23,5 Gew.-% einer Mischung aus 2 bis 4 gefällten Silikaten, 0,05 bis 0,5 Gew.-% wenigstens eines Viskositätsverstärkers, wenigstens eine oberflächenaktive Substanz, vorzugsweise mit einem Anteil von 0,2 bis 4,0 Gew.-% der einen oder der mehreren oberflächenaktiven Substanz(en), und mindestens 10 Gew.-% Wasser,
- Auftragen/Aufbringen der Zusammensetzung auf einen Mundeinsatz oder mehrere Mundeinsätze, der bzw. die für die Reinigung mehrerer, bevorzugt aller Zähne eines Benutzers geeignet ist/sind,
- Einführen des Mundeinsatzes bzw. der Mundeinsätze mit der Zusammensetzung in den Mund des Benutzers und
- Reinigen mehrerer, bevorzugt aller Zähne des Benutzers mit dem Mundeinsatz bzw. den Mundeinsätzen und der Zusammensetzung. Das Reinigen erfolgt vorzugsweise dadurch, dass der Mundeinsatz oder die Mundeinsätze mittels einer Antriebsvorrichtung zur Vibration angeregt werden.

Die bereitgestellte Zahnpflege- und Zahnreinigungszusammensetzung weist vorzugsweise eine Viskosität von 200 - 30.000 m Pa s auf.

Für das erfindungsgemäße Verfahren ist die hierin beschriebene erfindungsgemäße Zahnpflege- und Zahnreinigungszusammensetzung besonders geeignet. Sie stellt nicht nur eine ausreichende Wirkstoffmenge zur Verfügung, sondern kann darüber hinaus besonders gleichmäßig und einfach auf dem Mundeinsatz oder den Mundeinsätzen verteilt werden. Auch wird eine Überdosierung, wie sie bei herkömmlichen Zahncremes, -pasten oder -gelen auftreten würde, vermieden.

Die bereitgestellte Zusammensetzung ist somit vorzugsweise die erfindungsgemäße Zahnpflege- und Zahnreinigungszusammensetzung, umfassend
- 15 bis 30 Gew-% Glycerin,
- optional wenigstens ein weiteres Feuchthaltemittel, neben Glycerin, wobei das wenigstens eine weitere Feuchthaltemittel vorzugsweise Sorbitol oder hydrogeniertes Stärkehydrolysat, das vorzugsweise einen Sorbitol-Anteil im Bereich von 65 bis 75 Gew.-% aufweist, ist oder umfasst,
- 10 bis 23,5 Gew.-% wenigstens eines gefällten Silikats,
- 0,2 bis 4,0 Gew.-% wenigstens einer oberflächenaktiven Substanz,
- 0,05 bis 0,5 Gew.-% wenigstens eines Viskositätsverstärkers, und
- mindestens 10 Gew.-% Wasser.

Die Zusammensetzung weist ohne Treibgas vorzugsweise eine dynamische Viskosität von 200 - 30.000 m Pa s auf. Noch mehr bevorzugt liegt die Viskosität der Zusammensetzung ohne Treibgas in einem Bereich von 400 - 10.000 m Pa s und noch mehr bevorzugt bei weniger als 10.000 m Pa s. So ist beispielsweise eine Viskosität im Bereich von 500 - 9.000 m Pa s bei 20°C, noch mehr bevorzugt.

Bezüglich der bevorzugten wesentlichen und optionalen Inhalts- und Hilfs- oder Zusatzstoffe sowie deren bevorzugter Anteilsbereiche wird ausdrücklich auf die vorstehenden Ausführungen im Zusammenhang mit der Zusammensetzung als solches Bezug genommen.

Das Aufbringen/Auftragen der Zusammensetzung auf den Mundeinsatz oder die Mundeinsätze erfolgt vorzugsweise als Schaum, vorzugsweise als Schaumspray, oder als Aerosol. Die Zusammensetzung kann dann auf dem Mundeinsatz oder den Mundeinsätzen noch weiter expandieren und ein Zahnreinigungsmousse ausbilden. Vorzugsweise weist der zunächst leicht aufgeschäumte Zahnpastastrang nach Applikation bei 20 °C und Normaldruck eine Expansionsrate eine Expansionsrate von mehr als 100 Vol.-% in 3 bis 5 Sekunden auf. Noch mehr bevorzugt liegt die Expansionsrate bei 20 °C und Normaldruck nach 3 bis 5 Sekunden in einem Bereich von 150 Vol.-% bis 1000 Vol.-%.

Durch diese Applikation der Zusammensetzung auf den Mundeinsatz oder die Mundeinsätze wird die gleichmäßige und sparsame Verteilung der Zusammensetzung für den Benutzer weiter erleichtert und es kann eine gute Reinigung und Pflege der Zähne und des Zahnfleisches sichergestellt werden. Zudem weist das Aerosol oder Schaum (direkt oder als Schaumspray) oder das Mousse eine gewisse Lebensdauer auf bevor er/es sich in seine niedrigviskose Grundzusammensetzung auflöst. In dieser Zeit kann der Mundeinsatz bzw. können die Mundeinsätze in den Mund des Benutzers eingeführt werden. Der auf den Mundeinsatz aufgetragene Schaum / Aerosol oder das Mousse wird dann durch den Raum zwischen den Zähnen und Mundeinsatz zerdrückt und gleichmäßig verteilt. Somit kann der Schaum oder das Aerosol sehr sparsam auf den Mundeinsatz oder die Mundeinsätze aufgetragen werden.

Das Aufbringen der Zusammensetzung erfolgt vorzugsweise mit Hilfe eines der hierin beschriebenen Spender durch den die erfindungsgemäße Zusammensetzung in einen Schaum, vorzugsweise ein Schaumspray, oder ein Aerosol überführt werden kann. Bezüglich bevorzugter Ausführungen für diesen Spender wird ausdrücklich auf die vorstehende Offenbarung verwiesen.

Das erfindungsgemäße Zahnreinigungsverfahren umfasst weiterhin das Einführen des Mundeinsatzes oder der Mundeinsätze mit der Zusammensetzung in den Mund eines Benutzers und das gleichzeitige Reinigen und Pflegen mehrerer, bevorzugt aller Zähne des Benutzers durch Vibration des Mundeinsatzes. Die exakten Ausführungen der Mund- und Zahnreinigung über unterschiedliche Reinigungsstrukturen an den Innenflächen des Mundeinsatzes, Vibrationsfrequenzen, Reinigungsprogramme und Anwendungsdauer sind in der vorliegenden Erfindung nicht beschränkt.

Ein erfindungsgemäß zur Zahnreinigung und -pflege verwendbarer Mundeinsatz kann zur Reinigung aller Zähne zwei U-förmige Wannen zur Aufnahme der Zähne aufweisen, wobei sich die beiden Wannen dem Gebiss des Benutzers passen und in entgegengesetztem Richtungen geöffnet sind. Der Mundeinsatz ist zumindest grob, vorzugsweise individuell, an die Gebiss- und Kieferform eines Benutzers angepasst und entsprechend dimensioniert. Würde eine zu niedrigviskose Zahnreinigungszusammensetzung verwendet werden, kann diese beim Einsetzen des Mundeinsatzes aus der unteren Wanne herausfließen oder sich am Außenrand der unteren Wanne sammeln, was das Reinigungsergebnis, insbesondere an den Kauflächen der Zähne des Unterkiefers, negativ beeinträchtigen kann. Ein Aerosol, Schaum oder Mousse verbleibt hingegen durch seine inhärente Festigkeit längere Zeit in der Wanne, auch wenn diese mit der Öffnung nach unten gedreht wird.

In einer bevorzugten erfindungsgemäßen Ausführungsform wird der Mundeinsatz bzw. werden die Mundeinsätze zum Reinigen der Zähne, der Zahnhälse und des Zahnfleisches im Mund des Benutzers durch einen Motor zu Schwingungen/ Vibrationen mit einer Frequenz im Bereich von 20 bis 700 Hz, bevorzugt 25 bis 600 Hz angeregt.

Das gleichzeitige Reinigen der mehreren, vorzugsweise aller Zähne des Benutzers, dauert vorzugsweise zwischen 2 Sekunden und 5 Minuten, noch bevorzugter liegt die Zahnputzdauer in einem Bereich von 3 Sekunden bis zu 4 Minuten. Alternativ liegt die Obergrenze der Putzdauer bei 2 Minuten, 1,5 Minuten oder 1 Minute.

In einer erfindungsgemäßen Ausführungsform weist der Mundeinsatz eine Form auf, die die Zähne eines Benutzers während der Benutzung umgibt, so dass während des Reinigungsvorgangs Kauflächen und Innen- und Außenflanken der Zähne jeweils einer Fläche des Mundeinsatzes gegenüberliegen, und wobei eine Vielzahl von Reinigungsstrukturen auf den den Zähnen zugewandten Seiten des Mundeinsatzes vorgesehen sind.

In einer erfindungsgemäßen Ausführungsform ist der Mundeinsatz individuell an die Gebissform des Benutzers angepasst. Diese Anpassung kann durch herkömmliche negativ-positiv-Abdrücke, Abscannen und anschließendes 3D-Drucken des Mundeinsatzes, oder andere geeignete Verfahren erfolgen. Bevorzugte Materialien für den Mundeinsatz sind biokompatible Kunststoffe, z.B. biokompatibles Polyamid.

In einer erfindungsgemäßen Ausführungsform ist der Mundeinsatz einteilig oder, jeweils für das Oberkiefer und das Unterkiefer, zweiteilig ausgebildet.

Die Fig. 1 zeigt eine mögliche Ausführungsform eines Mundeinsatzes zur Anwendung mit einer Zusammensetzung gemäß einer bevorzugten Ausführungsform der vorliegenden Offenbarung. Insbesondere zeigt Fig. 1 eine perspektivische Ansicht eines einteiligen Mundeinsatzes 10. Die nicht sichtbare untere Struktur des Mundeinsatzes ist der oberen im Wesentlichen identisch, wobei anpassungsbedingte Formabweichungen möglich sind. Die Wandstärke der Wände 12 des Mundeinsatzes 10 beträgt typischerweise 1 bis 2,5 mm. Die äußere Begrenzung der Wände 12 verläuft in der Regel 1 bis 3 mm oberhalb des Zahnfleischrandes. Der Abstand zwischen den Reinigungsflächen, d.h. denjenigen Flächen des Mundeinsatzes 10, die beim Reinigungsvorgang den Zahnflächen des Benutzers zugewandt sind, und den Zahnoberflächen beträgt 0 bis 5 mm in Abhängigkeit von den gewählten Reinigungsstrukturen 14. In einer bevorzugten Ausführungsform sind auf den Reinigungsflächen Reinigungsstrukturen 14 vorgesehen.

Der Mundeinsatz 10 der Ausführungsform in Fig.1 umfasst ein angebrachtes Kupplungselement bzw. einen Kupplungsabschnitt 20 zur Verbindung des Mundeinsatzes 10 mit einer Antriebsvorrichtung (nicht gezeigt). Die Antriebsvorrichtung ist grundsätzlich mit Antriebsvorrichtungen vergleichbar, wie sie aus dem Stand der Technik auf dem Gebiet der Schallzahnbürsten oder dergleichen bekannt sind. Die Kopplung mit der Antriebsvorrichtung erfolgt über eine passende Bohrung 26.

In Fig. 1 wird verdeutlicht, dass ein relativ genau an das Gebiss des Benutzers angepasster Mundeinsatz viele Ausbuchtungen (den Zähnen entsprechend) und Stege (den Zahnzwischenräumen und Kauflächen entsprechend) aufweist. Diese Unebenheiten erschweren die Nutzung mit einer herkömmlichen Zahncreme oder ähnlichen Präparaten deutlich. Entweder muss sehr viel Zahncreme aufgetragen werden, was die Anwendungskosten erhöht und Ressourcen verschwendet. Ein akkurates Verteilen einer zähflüssigen Zahncreme mit einer Bürste oder einem Spatel wäre außerdem sehr zeitaufwändig für den Anwender/Benutzer. Die Reinigungsstrukturen 14 (in Fig. 2 gezeigt und unten detailliert beschrieben) erzeugen zusätzlich feine Unebenheiten, die mit einer herkömmlichen Zahncreme kaum zu erreichen bzw. auszufüllen sind.

Fig. 2 zeigt eine Detailansicht von Reinigungsstrukturen 14 in Form von Reinigungselementen, die an der Oberfläche des Mundeinsatzes 10 aus Fig. 1 in den Bereichen vorgesehen sein können, die in die Mundhöhle einführt werden und insbesondere in den Aussparungen, die mit den Zähnen in Kontakt kommen. In diesem Fall handelt es sich jeweils um Reinigungselemente, die einstückig mit dem Mundeinsatz 10 an dessen Oberfläche gebildet sind. Im Bereich der Kauflächen sind in dieser Ausführungsform rautenförmige Reinigungselemente 32 vorgesehen, im Bereich der vorderen und hinteren Zahnflächen sind jeweils zylinderförmige Reinigungselemente 30 ausgebildet. Aufgrund der Rautenform sind die Reinigungselemente 32 härter als die zylinderförmigen Reinigungselemente 30, so dass im Bereich der Kauflächen eine intensivere Zahnputzwirkung erzielt wird. Selbstverständlich können die Reinigungselemente 30 an den vorderen und hinteren Zahnflächen verschieden ausgebildet sein. Ebenso könnten in jedem der in Fig. 2 gezeigten Bereiche, d. h. im Bereich der hinteren Zahnflächen, der Kauflächen und der vorderen Zahnflächen, auch andere Reinigungsstrukturen 14 vorgesehen sein, beispielsweise Gummierungsschichten und/oder Streifenbürsten.

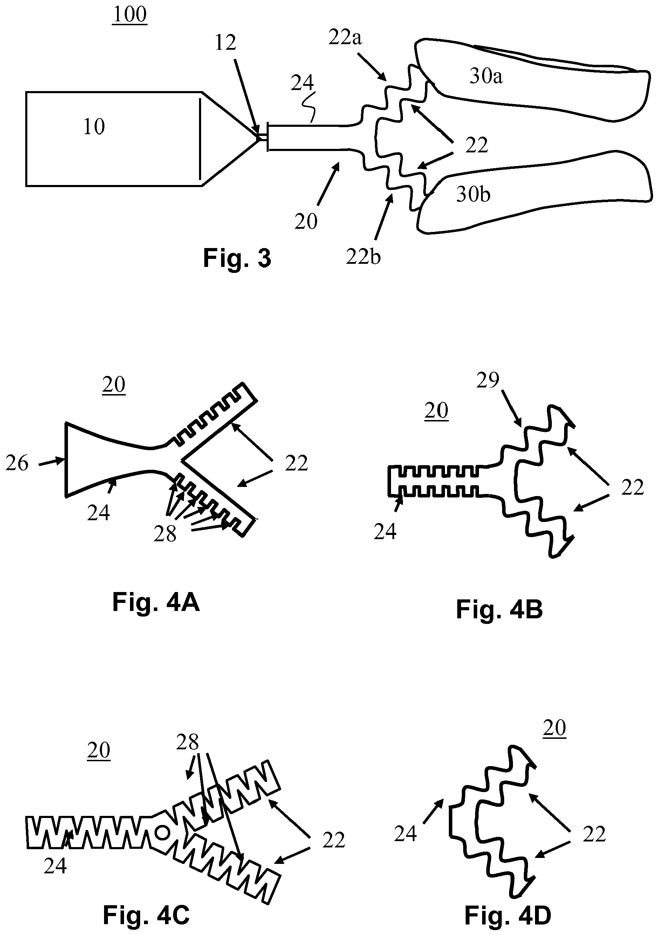

In den Figuren 3 bis 5 sind weitere Ausführungsformen eines Mundeinsatzes oder Teilen davon gezeigt, die besonders zur Anwendung in dem erfindungsgemäßen Verfahren bzw. gemäß der erfindungsgemäßen Verwendung geeignet sind. Der Mundeinsatz kann gemäß einer erfindungsgemäßen Ausführungsform des Verfahrens oder der erfindungsgemäßen Verwendung auch so ausgestaltet sein, dass er eine Trägerstruktur und eine Silikonhülle, die die Trägerstruktur im Wesentlichen vollständig umgibt, umfasst.

Aufgabe der Trägerstruktur ist es die Spannung des Mundeinsatzes zu erhalten und vor allem Vibrationen von einer Antriebsvorrichtung an die Zähne des Benutzers zu übertragen. Fig. 3 zeigt eine senkrechte Längsquerschnittsansicht einer erfindungsgemäß einsetzbaren Zahnputzvorrichtung 100. Die Trägerstruktur umfasst mindestens ein Mundeinsatzelement 30a für den Oberkiefer des Benutzers und mindestens ein Mundeinsatzelement 30b für den Unterkiefer des Benutzers. Das Mundeinsatzelement für den Ober- bzw. den Unterkiefer ist bevorzugt für alle Zähne des Ober- bzw. des Unterkiefers angepasst. Jedoch können die Mundeinsatzelemente auch mehrteilig ausgebildet sein, z. B. jeweils ein Abschnitt für die rechte und die linke Gebisshälfte des jeweiligen Kiefers oder jeweils ein Abschnitt für die Zahninnen-, Zahnaußen- und Kauflächenflanken. Die Ausführung mit jeweils genau einem Mundstück bzw. Mundeinsatzelement für den Ober- und den Unterkiefer ist jedoch bevorzugt. Die Mundeinsätze umfassen bevorzugt eine stabile Grundhalterung (im Folgenden auch als Basiskörper bezeichnet), die zur Schwingungskupplung mit einer Antriebsvorrichtung 10 dienen. Dieser Basiskörper umfasst im Wesentlichen ein biokompatibles Material, das bevorzugt die EN ISO 10993-1 erfüllt und/oder für den Lebensmittelkontakt gemäß der EU Kunststoff-Direktive 2002/72/EC zertifiziert ist. Besonders bevorzugt ist die Verwendung von biokompatiblem Polyamid (Homo- und/oder Copolyamid), wie etwa PA6, PA12, PA11, oder, besonders bevorzugt, PA6.6. In einer anderen Ausführungsform wird zur Fertigung der Basiskörper der Mundeinsätze Polyamid 12 verwendet, z.B. in der Ausführung PA 2200 (weiß) oder PA 2201 (transparent). Der gesamte Basiskörper kann einstückig aus dem oben beschriebenen biokompatiblen Material hergestellt sein.

Das Mundeinsatzelement für den Oberkiefer des Benutzers und das Mundeinsatzelement für den Unterkiefer können an deren Enden, die dem Kupplungsabschnitt 20 gegenüber liegen, miteinander verbunden sein. Damit sind die Abschnitte der Mundeinsatzelemente gemeint, die zur Reinigung der hinteren Seitenzähne vorgesehen sind.

Die Mundeinsatzelemente können so ausgestaltet sein, dass sie jeweils einen Trogförmigen oder U-förmigen Querschnitt aufweisen, der durch einen Trägerstrukturboden gebildet wird, der im Wesentlichen der Form der Bissfläche eines menschlichen Gebisses (Transversalebene des menschlichen Körpers) entspricht, eine Außenwand und eine Innenwand. Die Trägerstruktur kann aus Homo- oder Copolyamid, bevorzugt lebensmittelechtem Homo- oder Copolyamid, weiterhin bevorzugt aus PA 6, PA, 6.6, PA 4.6, PA 11, PA 12, PA 1010, PA 610, Copolyamiden oder Polyamidmischungen aus diesen, und besonders bevorzugt PA 6.6, Copolyamiden oder Polyamidmischungen mit diesem gefertigt sein.

Die Trägerstruktur umfasst in einer bevorzugten Form zudem einen Kupplungsabschnitt 20, der mindestens zwei Arme 22 und einen Antriebsvorrichtungsverbindungsabschnitt 24 aufweist, wobei die mindestens zwei Mundeinsatzelemente 30a und 30b jeweils mit einem Arm 22 des Kupplungsabschnitts 20 verbunden sind und der Antriebsvorrichtungsverbindungsabschnitt 24 angepasst ist, um mit der Antriebsvorrichtung 10 verbunden zu werden.

In anderen bevorzugten Formen können die Mundeinsatzelemente jeweils auch durch zwei oder mehr Arme mit dem Antriebsvorrichtungsverbindungsabschnitt 24 verbunden sein. Die Zahnputzvorrichtung 100 zeichnet sich in einer Ausführungsform dadurch aus, dass die Arme 22 des Kupplungsabschnitts 20, die mit den Mundeinsatzelementen 30a und 30b verbunden sind, jeweils mindestens einen Federabschnitt 22a oder 22b aufweisen. Die Federabschnitte 22a und 22b weisen bevorzugt eine geringere Steifigkeit, als die übrigen Bereiche der Arme 22 bzw. der Armbereiche ohne Federabschnitte, und eine hohe Flexibilität auf, so dass die Federabschnitte die Mundeinsatzelemente 30a und 30b bei Belastung in Richtung einer Grundstellung vorspannen. Die Federabschnitte 22a und 22b ermöglichen zum einen ein leichteres Einsetzen der Mundeinsatzelemente in den Mundraum. Zudem werden Eigenresonanzschwingungen der Mundeinsatzelemente 30a und 30b zueinander durch den gefederten Kupplungsabschnitt 20 gedämpft. Es sei angemerkt, dass sich die Federabschnitte 22a und 22b in einigen Ausführungsformen über die gesamt Länge eines Arms 22 erstrecken können. Bevorzugt erstrecken sich die Federabschnitte über 20% bis 100% der Länge eines Arms 22 und insbesondere über 35% bis 95% oder besonders bevorzugt 65% bis 90% des Arms 22. Die Federabschnitte 22a und 22b des Kupplungsabschnitts 20 bewirken weiterhin eine verbesserte Schwingungsübertragung auf die Zähne des Benutzers.

Der Kupplungsabschnitt 20 kann mit den Federabschnitten einstückig ausgebildet sein. Ebenso können die Federabschnitte einstückig mit den Mundeinsatzelementen und/oder den nicht gefederten Abschnitten des jeweiligen Arms ausgebildet sein. Die einstückige Ausbildung verbessert die Schwingungsübertragung zwischen Antriebsvorrichtung 10 und Mundstück. Zudem werden Zwischenräume vermieden, die den Reinigungsaufwand erhöhen oder die hygienischen Bedingungen des Geräts verschlechtern könnten. Es ist bevorzugt, dass der Kupplungsabschnitt 20 zur besseren Kontrolle des Eigenresonanzverhaltens aus dem identischen Material besteht, bevorzugt biokompatiblen Polyamid, besonders bevorzugt PA 6.6 oder PA 12 wie PA 2200 oder PA 2201, wie die Basisteile der Mundeinsätze.

Ebenso können Trägerstrukturen verwendet werden, bei denen die Federabschnitte über Steck- oder Schraubverbindungen austauschbar sind. Über einen solchen modularen Aufbau des Kupplungsabschnitts 20 können Wartungen ermöglicht werden, die den Ersatz des gesamten Kupplungsabschnitts 20 unnötig machen können.

Die Federabschnitte können im Vergleich zu dem jeweiligen Arm des Kupplungsabschnitts 20, mit dem sie verbunden sind, verjüngt und geschwungen bzw. S-förmig auf und ab gebogen angeordnet. Die Verjüngung der Kupplungsabschnitte kann dabei durch Plattdrücken (z.B. unter Wärme- und Druckeinwirkung oder Walzen) der jeweiligen Arme des Kupplungsabschnitts gebildet sein, und die S-Form kann durch anschließendes Auf- und Abbiegen der plattgedrückten Arme, in der Ebene, die längs durch die Arme 22 und den Antriebsvorrichtungsverbindungsabschnitt 24 des Kupplungsabschnitts 20, also in der senkrechten Symmetrieebene des Gebisses des Anwenders, verläuft, gebildet werden.

Auch können die Federabschnitte durch mehrere Einkerbungen an den Außenseiten und/oder den Innenseiten jeweils an den Armen des Kupplungsabschnitts ausgebildet sein. Bevorzugt können die Federabschnitte durch Einschnitte an den Außenseiten der Arme des Kupplungsabschnitts oder abwechselnd und versetzt zueinander jeweils an den Außen- und Innenseiten des Kupplungsabschnitts gebildet sein, die bezüglich der Längsachse des jeweiligen Arms senkrecht verlaufen und mindestens bis zur Hälfte des jeweiligen Arms eingeschnitten sind. In einer besonders bevorzugten Ausführungsform ist die Trägerstruktur jedoch direkt durch Spritzguss oder Vakuumspritzguss gefertigt, so dass eine Nachbehandlung durch Einschnitte bzw. Erwärmen und Plattdrücken zwar möglich, jedoch nicht notwendig ist. Stattdessen kann die Form der Federabschnitte direkt durch entsprechende Ausbildung der Gussform festgelegt werden.

Der Längsquerschnitt durch die drei Arme kann dabei bevorzugt Y-Form aufweisen. Alternative Querschnitte sind Stimmgabelform oder, ohne den dritten Arm, U-Form oder Hufeisen-Form. Anstatt des dritten Arms kann der Antriebsvorrichtungskupplungsabschnitt 24 auch direkt an der Stelle vorgesehen sein, an dem die zwei Arme 22, die mit den Mundstücken verbunden sind, zusammentreffen.

In Fig. 4A ist eine senkrechte Längsquerschnittsansicht eines bevorzugten Kupplungsabschnitts 20 gezeigt. Der Kupplungsabschnitt 20 weist zwei Arme 22 auf, die jeweils einen Federabschnitt umfassen. Die Federabschnitte sind durch mehrere Trog-förmige Einschnitte 28 an den Außenflanken der Arme 22 des Kupplungsabschnitts 20 ausgebildet. Die Kanten der Einschnitte sind bevorzugt abgerundet. Alternative Einschnittformen (V-förmig, U-förmig etc.) sind ebenfalls möglich.

Vorzugsweise besteht der Kupplungsabschnitt 20 zur besseren Kontrolle des Eigenresonanzverhaltens aus dem gleichen Material, wie die Trägerstrukturen bzw. der Basiskörper der Mundeinsatzelemente 30a, 30b.

Fig. 4B zeigt eine weitere Gestaltungsform eines Kupplungsabschnitts 20. Hier sind die Arme 22 mit federnden Abschnitten 29 versehen, die als in der senkrechten Ebene auf und ab gebogene Mäander ausgebildet sind. Andere Ausbildungen der Mäander, z.B. in der waagerechten Ebene (Transversalebene des menschlichen Körpers bzw. senkrecht zur dargestellten Bildebene) oder als Helix- bzw. Spiralförmig gebogene Elemente, sind möglich, jedoch nicht explizit dargestellt. Die Federabschnitte sind bevorzugt im Vergleich zu den Armen 22 ohne Federabschnitte verjüngt. In einer Ausführungsform beträgt die Stärke der Arme im Bereich der Federabschnitte 10 bis 50 %, bevorzugt 20 bis 40 % der Stärke der Arme ohne die Federabschnitte.

Der Antriebsvorrichtungsverbindungsabschnitt 24 kann optional ebenfalls Federabschnitte umfassen oder, wie in Fig. 4B beispielhaft dargestellt, durch einen Federabschnitt, der sich zwischen Antriebsvorrichtung und Verbindungsstelle der Arme 22 erstreckt, bereitgestellt sein. Hier sind diese an gleichen Positionen der Ober- und der Unterseite des Antriebsvorrichtungsverbindungsabschnitts 24 vorgesehen. Entsprechende Federabschnitte können auch als die Federabschnitte 22a, 22b vorgesehen sein.

Fig. 4C zeigt eine weitere Ausgestaltungsform eines Kupplungsabschnitts 20. Hier sind die Arme 22 und der Antriebsvorrichtungsverbindungsabschnitt 24 mit dem gleichen Federungsmuster versehen, das durch Einschnitte 28 abwechselnd an der Ober- und Unterseite der Arme 22 und des Antriebsvorrichtungsverbindungsabschnitts 24 gebildet ist. Die Einschnitte können, wie in Fig. 4C gezeigt, V-förmig sein oder eine andere Form ausweisen.

Fig. 4D zeigt eine weitere Ausgestaltungsform eines Kupplungsabschnitts 20. Hier sind die Arme 22, ähnlich der Fig. 3B, durch auf und ab biegen der verjüngten Arme 22 gebildet. Jedoch ist der Antriebsvorrichtungsverbindungsabschnitt 24 nicht als zusätzlicher Arm, sondern als ein einfaches Anbindungsmittel, beispielsweise als Öffnung, Bohrung oder Stift, an der Position vorgesehen, an der die Arme 22 zusammenlaufen. Diese Ausführungsform des Antriebsvorrichtungsverbindungsabschnitts 24 ist mit den Ausbildungen der Arme gemäß Fig. 4A bis 4C uneingeschränkt kompatibel.

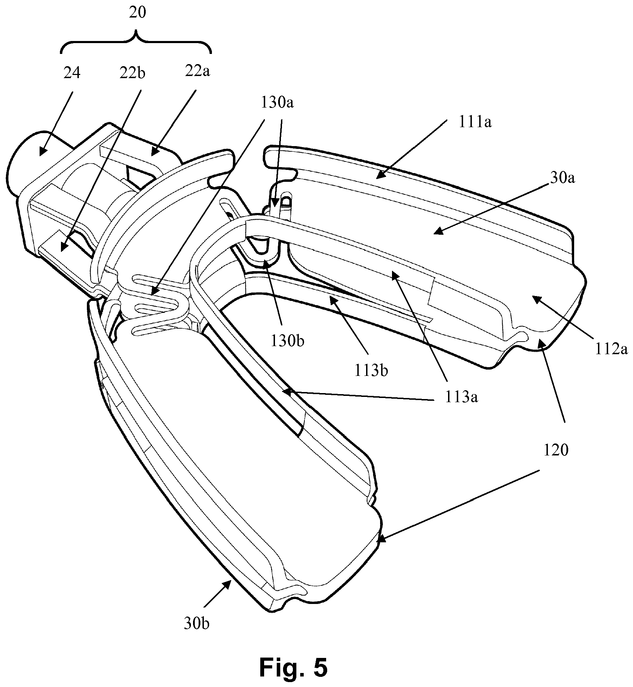

Fig.5 zeigt eine r Modifikation der ersten bevorzugten Ausgestaltung des Mundeinsatzes. Hierbei umfasst wenigstens eines der Mundeinsatzelemente 30a oder 30b mindestens einen ersten Abschnitt und einen zweiten Abschnitt, die mit einem Federabschnitt bzw. Schwenkfederabschnitt 130a oder 130b miteinander verbunden sind. Der Schwenkfederabschnitt liegt in der Gebissebene (Transversalebene des menschlichen Körpers), die sich im Wesentlichen parallel zu und zwischen den Mundeinsatzelementen 30a und 30b erstreckt, weist eine geringere Steifigkeit auf, als der erste und zweite Abschnitt, um eine Verformung des jeweiligen Mundeinsatzelements 30a oder 30b in der Gebissebene zu erleichtern. Als Gebissebene ist dabei die Ebene definiert, die den Kauflächen der Zähne des Benutzers entsprechenden Seiten der Mundeinsatzelemente 30a und 30b entsprechen.

Bei dieser Ausgestaltung weisen die Außenwände 111a jeweils im Bereich der Schwenkfederabschnitte 130a und 130b eine Aussparung auf, die durch Vibrationskopplungsmechanismus überbrückt ist. Die Vibrationskopplungsmechanismen umfassen optional jeweils einen ersten Kopplungsabschnitt und einen zweiten Kopplungsabschnitt, die an gegenüberliegenden Seiten der Aussparung der Außenwand angeordnet sind, sich zueinander erstrecken und einander berühren.

Das Mundeinsatzelement 30a für den Oberkiefer des Benutzers und das Mundeinsatzelement für den Unterkiefer 30b können an deren Enden 120, die dem Kupplungsabschnitt 20 gegenüber liegen, miteinander verbunden sein.

Gemäß dieser Ausgestaltung kann die Trägerstruktur auch ein Mundeinsatzelement für den Oberkiefer des Benutzers und ein Mundeinsatzelement für den Unterkiefer des Benutzers und einen Kupplungsabschnitt umfassen, der zwei Arme und einen Antriebsvorrichtungsverbindungsabschnitt 24 aufweist, wobei die Mundeinsatzelemente jeweils mit einem Arm des Kupplungsabschnitts verbunden sind und der Antriebsvorrichtungsverbindungsabschnitt angepasst ist, um mit einer Antriebsvorrichtung über deren Trägerstruktur verbunden zu werden. Diese Ausgestaltungsform unterscheidet sich von der ersten Ausgestaltungsform dadurch, dass die Trägerstruktur nur optional Arme mit Federabschnitten aufweist und stattdessen wenigstens eines der Mundeinsatzelemente wenigstens einen ersten Abschnitt und einen zweiten Abschnitt umfasst, die mit einem Schwenkfederabschnitt verbunden sind, der in der Gebissebene, die sich im Wesentlichen parallel zu und zwischen den Mundeinsätzen erstreckt, eine geringere Steifigkeit aufweist, als der erste und zweite Abschnitt, um eine Verformung des jeweiligen Mundeinsatzelements in der Gebissebene zu erleichtern. Mit anderen Worten müssen die Arme des Kupplungsabschnitts der Trägerstruktur der zweiten Ausführungsform nicht mit den jeweils mindestens einen Federabschnitt ausgestattet sein. Es ist jedoch ausdrücklich vorteilhaft und daher wünschenswert, dass die erste und die zweite Ausführungsform miteinander kombiniert werden.

Entsprechend kann die Trägerstruktur der zweiten Ausgestaltungsform ebenfalls solche Arme des Kupplungsabschnitts 20 aufweisen, die mit den Mundeinsatzelementen verbunden sind und die jeweils mit mindestens einem Federabschnitt vorgesehen sind. Die Federwege der Federabschnitte der Arme 22 der Trägerstruktur der ersten bevorzugten Ausführungsform (in der Sagittalebene des menschlichen Körpers) sind im Wesentlichen senkrecht zur dem Federweg des Schwenkfederabschnitts 130a, 130b der zweiten bevorzugten Ausführungsform (in der Transversalebene des menschlichen Körpers).

Bei der Trägerstruktur einer bevorzugten Form umfassen die Mundeinsatzelemente jeweils einen Basiskörper aus biokompatiblem Material, bevorzugt biokompatiblem Polyamid, der grob an eine Form der Zähne des Unterkiefers oder Oberkiefers eines Benutzers angepasst ist. Der Basiskörper ist für die Weiterleitung der Vibrationen hin zu den Reinigungsflächen verantwortlich und besteht in einer Ausführungsform vollständig aus einem biokompatiblen Polyamid. Besonders bevorzugt ist die Verwendung von biokompatiblem Polyamid, wie etwa PA6, PA6.6, PA11, oder PA12, bevorzugt PA 6.6. In einer Ausführungsform wird zur Fertigung der Basiskörper der Mundeinsatzelemente Polyamid 12 verwendet, z.B. in der Ausführungsform PA 2200 (weiß) oder PA 2201 (transparent). Dem Polyamid können optional Additive, Farbstoffe, und/oder verstärkende Fasern beigefügt werden.

Außerdem können die Mundeinsatzelemente einen an den Innenseiten des Basiskörpers, also an den während der Benutzung der Vorrichtung den Zähnen des Benutzers zugewandten Seiten, befestigten (z.B. über Einkleben, Eindrücken oder eine Art Klettverbindung) Einsatzkörper aus einem thermoplastischen Material umfassen, der passgenau an eine Form der Zähne des Unterkiefers oder Oberkiefers eines Benutzers angepasst ist. Als thermoplastisches Material wird in einer Ausführungsform ein thermoplastisches Elastomer verwendet. Bevorzugt sind dabei Polycaprolacton (PCL), z. B. Polydoh^{®} der Firma Polymorph, thermoplastisches Polyesterelastomer TPE-E, z.B. Keyflex^{®} der Firma LG Chemie, thermoplastisches Elastomer auf Urethanbasis TPE-U z. B. Elastollan^{®} (BASF), oder Mischungen aus diesen Stoffen. Additive oder verstärkende Fasern können diesen Materialien optional zugefügt werden.

Die Trägerstruktur kann so ausgestaltet sein, dass die Einsatzkörper jeweils an ihren Innenflächen, also den Zähnen des Benutzers zugewandten Reinigungsflächen, eine Vielzahl von Reinigungsstrukturen aufweisen. Die Reinigungsstrukturen können Gummierungsschichten, die auf den Innenflächen des Einsatzkörpers angebracht sind, Reinigungselemente, die einstückig mit Oberflächenbereichen des Einsatzkörpers ausgebildet sind und/oder Streifenbürsten, die auf den Innenflächen des Einsatzkörpers angebracht sind, umfassen. Beispiele und genauere Beschreibungen der Reinigungsstrukturen sowie deren Implementierung sind in der DE 102015109891 A1, Fig. 4 und in deren Absatz [0050] beschrieben. So können (mit Bezug auf Fig. 4 der DE 102015109891 A1, wobei diese Figur hierin unter Bezugnahme aufgenommen ist) die Reinigungsstrukturen in Form von Reinigungselementen, die an der Oberfläche des Mundeinsatzes, dessen Silikonhülle oder den Oberflächen der Mundeinsatzelemente vorgesehen sein können, ausgebildet sein. Die Reinigungselemente können einstückig mit dem Mundeinsatz an dessen Oberfläche oder einstückig mit der Silikonhülle gebildet sein. Im Bereich der Kauflächen sind in dieser Ausführungsform rautenförmige Reinigungselemente vorgesehen, im Bereich der vorderen und hinteren Zahnflächen jeweils zylinderförmige Reinigungselemente. Aufgrund der Rautenform sind die Reinigungselemente härter als die zylinderförmigen Reinigungselemente, so dass im Bereich der Kauflächen eine intensivere Zahnputzwirkung erzielt wird. Selbstverständlich können die Reinigungselemente an den vorderen und hinteren Zahnflächen verschieden ausgebildet sein. Ebenso könnten in jedem der gezeigten Bereiche, d.h. im Bereich der hinteren Zahnflächen, der Kauflächen und der vorderen Zahnflächen, auch andere Reinigungsstrukturen vorgesehen sein, beispielsweise Gummierungsschichten und/oder Streifenbürsten.

In den vorstehend beschriebenen Trägerstrukturen sind die Mundeinsatzelemente optional jeweils für die Reinigung der Zähne des gesamten Oberkiefers oder des gesamten Unterkiefers des Benutzers angepasst.

Insbesondere durch eine variierende Formgebung der Reinigungsstrukturen wird die Nutzung herkömmlicher Zahncremes zusätzlich erschwert. Dringt eine bestimmte Zahncreme beispielsweise gut in den Raum zwischen den rautenförmigen Elementen 32 ein, so sind die Abstände zwischen den Borstenstrukturen 30 zu gering, um eine ausreichende und einfache Verteilung der Zahncreme zu ermöglichen und bei unachtsamer Anwendung kann sich das Reinigungsergebnis verschlechtern.

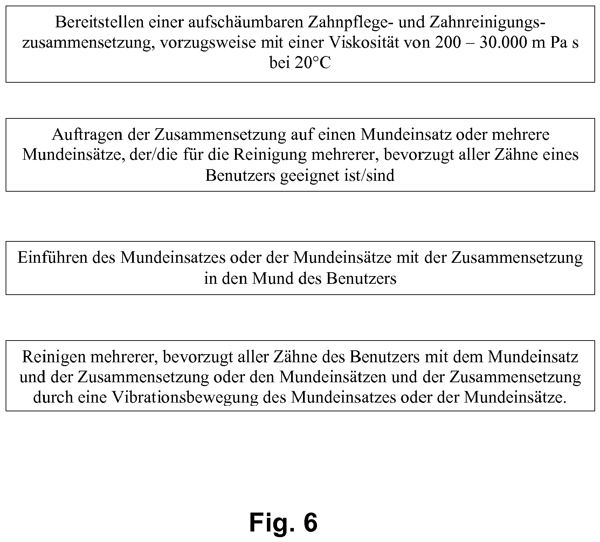

Das durch die vorliegende Offenbarung bereitgestellte erfindungsgemäße Verfahren zum Reinigen und Pflegen der Zähne und/oder des Zahnfleisches eines Benutzers, insbesondere zum Vorbeugen und/oder Behandeln von Erkrankungen der Zähne, der Zahnhälse und/oder des Zahnfleisches mit einer , vorzugsweise ebenso erfindungs-gemäßen, Mund-, Zahnpflege- und Zahnreinigungs-zusammensetzung wird in Fig. 6 zusammengefasst.

Das erfindungsgemäße Verfahren zum Reinigen und Pflegen der Zähne und/oder des Zahnfleisches sollte vorzugsweise mindestens zweimal am Tag, noch mehr bevorzugt zwei bis viermal pro Tag, beispielsweise zweimal oder dreimal pro Tag, durchgeführt werden.

Wie bereits vorstehend im Detail erläutert, betrifft die vorliegende Erfindung gemäß dem ersten Aspekt eine Zahnpflege- und Zahnreinigungszusammensetzung zur Verwendung in einem Verfahren zum gleichzeitigen Reinigen mehrerer, vorzugsweise aller Zähne des Ober- und/oder Unterkiefers, mittels einer hierfür geeigneten Zahnputzvorrichtung, wobei die Zahnpflege- und Zahnreinigungszusammensetzung in aufgeschäumter Form auf einem Mundeinsatz oder mehreren Mundeinsätzen der Zahnputzvorrichtung dargereicht wird.

Die gemäß diesem ersten Aspekt der vorliegenden Erfindung eingesetzt Mund-, Zahnpflege- und Zahnreinigungszusammensetzung ist vorzugsweise die erfindungsgemäße Zahnpflege- und Zahnreinigungszusammensetzung gemäß dem zweiten Aspekt der vorliegenden Erfindung. Die bevorzugte Zusammensetzung umfasst:
- 15 bis 30 Gew.-%, vorzugsweise mindestens 20 Gew.-%, noch mehr bevorzugt mindestens 22 Gew.-%, Glycerin,
- optional wenigstens ein weiteres Feuchthaltemittel, neben Glycerin, wobei das wenigstens eine weitere Feuchthaltemittel vorzugsweise Sorbitol oder hydrogeniertes Stärkehydrolysat, das vorzugsweise einen Sorbitol-Anteil im Bereich von 65 bis 75 Gew.-% aufweist, ist oder umfasst,
- 10 bis 23,5 Gew.-% wenigstens eines gefällten Silikats,
- 0,2 bis 4,0 Gew.-% wenigstens einer oberflächenaktiven Substanz,
- 0,05 bis 0,5 Gew.-% wenigstens eines Viskositätsverstärkers, und
- mindestens 10 Gew.-% Wasser.

Bevorzugte Ausführungsformen des Verfahrens und der Verwendung sind oben im Zusammenhang mit der Offenbarung zu der Zusammensetzung beschrieben. Da diese identisch auf das Verfahren und die Verwendung der Zusammensetzung übertragen werden können, wird hierauf Bezug genommen.

Die Anwendung einer niedrigviskosen Zusammensetzung gemäß der vorliegenden Offenbarung ermöglicht das leichte und fehlerfreie Auftragen auf einen Mundeinsatz. Durch das bevorzugte Auftragen der Zusammensetzung in Form eines Schaums, noch mehr bevorzugt in Form eines Schaumsprays, wird insbesondere eine verbesserte Haftung auf den Reinigungsflächen in Fig. 1 gewährleistet, die nicht gezeigt, also nach unten gerichtet sind.

Ebenso betrifft die vorliegende Erfindung die Verwendung der hierin beschriebenen Zusammensetzung zur Verwendung im Bereich der Mund- und Zahnhygiene. Bevorzugt wird die Zusammensetzung mittels einer Zahnputzvorrichtung für die gleichzeitige Reinigung mehrerer, vorzugsweise aller Zähne eines Benutzers appliziert. Die Zusammensetzung wird gemäß dieser bevorzugten Ausführungsform dargereicht durch Aufbringen der Zusammensetzung auf einen Mundeinsatz oder auf mehrere Mundeinsätze der Zahnputzvorrichtung.

### Kurze Beschreibung der Figuren

Fig. 1 zeigt eine Ausführungsform eines Mundeinsatzes einer Zahnputzvorrichtung, der zur gleichzeitigen Reinigung aller Zähne eines Benutzers und zur Applikation der erfindungsgemäßen Zusammensetzung geeignet ist. Der Mundeinsatz umfasst einen Kupplungsabschnitt zur Verbindung des Mundeinsatzes mit einer Antriebsvorrichtung der Zahnputzvorrichtung (nicht gezeigt).
Fig. 2 zeigt eine Aufsicht auf einen Teil eines Mundeinsatzes mit darin enthaltenen Reinigungsstrukturen.
Fig. 3 ist eine senkrechte Längsquerschnittsansicht einer Zahnputzvorrichtung zum gleichzeitigen Reinigen aller Zähne eines Benutzers im Sinne der vorliegenden Erfindung.
Fig. 4A bis 4D sind jeweils senkrechte Längsquerschnittsansichten von Kupplungsabschnitten unterschiedlicher Ausführungsformen für einen Mundeinsatz einer Zahnputzvorrichtung, der zur gleichzeitigen Reinigung aller Zähne eines Benutzers geeignet ist.
Fig. 5 ist eine Perspektivansicht einer Trägerstruktur für eine andere Ausführungsform eines Mundeinsatzes einer Zahnputzvorrichtung, der zur gleichzeitigen Reinigung aller Zähne eines Benutzers geeignet ist.
Fig. 6 ist ein Flussdiagramm eines Verfahrens zum gleichzeitigen Reinigen mehrerer, vorzugsweise aller Zähne eines Benutzers.
Fig. 7A bis 7F zeigen fotografische Aufnahmen, in Negativdarstellung, zur Schaumstabilität einer erfindungsgemäßen Zusammensetzung (jeweils links) im Vergleich zu einer Zusammensetzung mit niedrigem Glycerin- und Wassergehalt (jeweils rechts) über die Zeit. Fig. 7A zeigt die Schäume zum Zeitpunkt 0 Sekunden, Fig. 7B zeigt die Schäume zum Zeitpunkt 30 Sekunden, Fig. 7C zeigt die Schäume zum Zeitpunkt 1 Minute, Fig. 7D zeigt die Schäume zum Zeitpunkt 2 Minuten, Fig. 7E zeigt die Schäume zum Zeitpunkt 3,5 Minuten und Fig. 7F zeigt die Schäume zum Zeitpunkt 5 Minuten.
Fig. 8 stellt die Abnahme des Schaumvolumens in Abhängigkeit der Zeit dar. Verglichen werden eine erfindungsgemäße Zusammensetzung (als "Erfindung" gekennzeichnet) und eine Zusammensetzung mit niedrigem Glycerin- und Wassergehalt (als "Referenz" gekennzeichnet).

### Messmethoden:

Im Rahmen der vorliegenden Erfindung erfolgt die Bestimmung der Viskosität mit einem Brookfield Viskosimeter, vorzugsweise einem Rotationsviskosimeter DV2T der Firma Brookfield mit Heliopath T-Spindeln vom Spindeltyp C. Die Messung erfolgt bei einer Drehzahl von 10 rpm (Umdrehungen pro Minute), 20 °C und Normaldruck von 0,1 MPa (1013,25 mbar).

Durch Messung des zur Drehung der Spindel benötigten Drehmoments über die Auslenkung einer Feder wird die Viskosität der erfindungsgemäßen Zusammensetzung ermittelt.

Bestimmt wird die Viskosität der flüssigen Zusammensetzung ohne Treibgas, also zu einem Zeitpunkt bevor die Zusammensetzung gemäß einer bevorzugten Ausführungsform in einen Schaum oder ein Aerosol überführt wird.

Die vorstehend angegebenen Viskositäten oder Viskositätsbereiche meinen also jeweils die mit einem Brookfield Viskosimeter, vorzugsweise einem Rotationsviskosimeter DV2T der Firma Brookfield, mit Heliopath T-Spindeln vom Spindeltyp C bei einer Drehzahl von 10 rpm (Umdrehungen pro Minute), 20 °C und Normaldruck von 0,1 MPa (1013,25 mbar) gemessenen dynamischen Viskositäten in der Zusammensetzung ohne Treibgas.

Die Bestimmung der spezifischen Oberfläche in (m²/g) nach der BET-Methode durch die Adsorption von Gasen, beispielsweise von Stickstoff N₂, erfolgt nach Brunauer, Emmett und Teller gemäß DIN 66131.

Die Bestimmung der mittleren Teilchengröße der Silikate erfolgt unter Verwendung eines Leeds and Northrup Microtrac II. Ein Laserstrahl wird durch eine transparente Zelle projiziert, die einen Strom sich bewegender Partikel, die in einer Flüssigkeit suspendiert sind, enthält. Lichtstrahlen, die die Partikel treffen, werden um Winkel gestreut, die umgekehrt proportional zu ihrer Größe sind. Die Photodetektor-Anordnung misst die Lichtmenge bei mehreren vorbestimmten Winkeln. Elektrische Signale, die proportional zu den gemessenen Lichtstromwerten sind, werden dann von einem Mikrocomputersystem verarbeitet, um ein Mehrkanal-Histogramm der Partikelgrößenverteilung zu bilden.

Die Bestimmung des RDA-Werts erfolgt nach der in dem Artikel "A Laboratory Method for Assessment of Dentifrice Abrasivity", John J. Hefferren, im Journal of Dental Research, Vol. 55, Nr. 4 (1976), Seiten 563-573 beschriebenen Methode.

Der Messing-Einlehner-(BE)-Abrasionswert bzw. die Einlehner Abrasion wurde durch die Verwendung einer Einlehner AT-1000 Abriebprüfmaschine gemessen. In dieser Prüfung wird ein Fourdrinier-Messingdrahtsieb gewogen und der Wirkung einer 10%igen wässrigen Silikatsuspension für eine bestimmte Anzahl an Umdrehungen ausgesetzt und die Abriebmenge dann als Milligramm Materialverlust von dem Fourdrinier-Drahtsieb pro 100.000 Umdrehungen bestimmt. Das Ergebnis ist der 10% Messing-Einlehner-(BE)-Abrasionswert.

Der Drehkörper der Abriebprüfmaschine läuft unabhängig von der Netzfrequenz (50/60 Hz) einheitlich mit 1800 Umdrehungen/Minute. Die Silikatsuspension wird durch Mischen des zu testenden Silikats mit entionisiertem Wasser hergestellt. Zur Bereitstellung einer 10%igen Suspension werden beispielsweise 100 g Silikat mit 900 g Wasser gemischt.

### BEISPIELE

### Beispiel 1: Zahnpflegezusammensetzung

In einem Vakuumhomogenisator werden bei 6000 Umdrehungen pro Minute folgende Schritte zur Herstellung einer Zahnpflegegrundformulierung durchgeführt:
1. Vorlage von 1500 g hydrogeniertem Stärkehydrolysat mit einen Sorbitol-Anteil von 70%
2. Zugabe von 100 g Olaflur (aminfluoridhaltiger Rohstoff mit einem Wirkstoffanteil von 35 %)
3. Zugabe von 2000 g oder 1700 g Glycerin
4. Homogenisieren des so erhaltenen Gemisches für 10 min
5. Zugabe von 500 g Zeodent 115
6. Zugabe von 300 g Zeodent 167
7. Zugabe von 500 g Zeodent 103
8. Homogenisieren des so erhaltenen Gemisches für 10 min
9. Zugabe von 6 g Xanthan Gum
10. Zugabe von 4 g Acrylat Crosspolymer C10-C30
11. Homogenisieren des so erhaltenen Gemisches für 10 min
12. Zugabe von 50 g Natriumlaurylsulfat
13. Zugabe von 10 g Cocamidopropylbetain
14. Homogenisieren des so erhaltenen Gemisches für 10 min
15.Zugabe von Wasser ad 6000g
16. Einstellung pH=7,0 mit Natriumhydroxid

Während des Abfüllprozesses der so erhaltenen Zusammensetzung in einen Beutel einer Bag-on-Valve Dose werden der Zusammensetzung noch 4,9 Gew.-% kosmetischer Schaumbildner (Treibgas), bezogen auf die treibgashaltige Zusammensetzung, hinzugefügt.

Die vorstehend genannte, beispielhafte Zusammensetzung kann in den Mundstücken manuell durch Aufsprühen als Schaum oder Aerosol in diesen verteilt werden. Es hat sich gezeigt, dass sich die Zusammensetzung sehr gleichmäßig in den Aussparungen des Mundstücks verteilt.

Es hat sich außerdem gezeigt, dass insbesondere der Putzkörper, aber auch die anderen Aktivstoffe gleichmäßig in dem Schaum oder Aerosol verteilt sind. Damit ist sichergestellt, dass lokale Überdosierungen oder Unterdosierungen vermieden werden. Somit konnten mit der erfindungsgemäßen Zusammensetzung sehr gute Reinigungsergebnisse erzielt werden.

Die beispielhafte Zusammensetzung hat sich außerdem dadurch vorteilhaft ausgezeichnet, dass sie und der aus ihr gebildete Schaum gut und ausreichend lange an dem Mundstuck haftet, um ein Einführen des Mundstücks in die Mundhöhle zu ermöglichen, ohne dass sie oder der Schaum aus dem Mundstuck herausläuft. Das galt insbesondere auch für die Zusammensetzung bzw. den Schaum der sich in der nach unten geöffneten Aussparung für die Aufnahme der Zähne des Unterkiefers, also in Kopfüberlage, befand. Im Mund zerfällt der Schaum aber wieder.

Die Anhaftung (Adhäsion) ist messbar mit einem Tensiometer.

### Beispiel 2: Zahnpflegezusammensetzung

In einem Vakuumhomogenisator werden bei 6000 Umdrehungen pro Minute folgende Schritte zur Herstellung einer Zahnpflegegrundformulierung durchgeführt:
1. Vorlage von 1500 g Sorbitol
2. Zugabe von 100 g Olaflur (aminfluoridhaltiger Rohstoff mit einem Wirkstoffanteil von 35 %)
3. Zugabe von 1700 g Glycerin
4. Homogenisieren des so erhaltenen Gemisches für 10 min
5. Zugabe von 500 g Zeodent 115
6. Zugabe von 300 g Zeodent 167
7. Zugabe von 500 g Zeodent 103
8. Homogenisieren des so erhaltenen Gemisches für 10 min
9. Zugabe von 6 g Xanthan Gum
10. Zugabe von 4 g Acrylat Crosspolymer C10-C30
11. Homogenisieren des so erhaltenen Gemisches für 10 min
12. Zugabe von 50 g Natriumlaurylsulfat
13. Zugabe von 10 g Cocamidopropylbetain
14. Homogenisieren des so erhaltenen Gemisches für 10 min
15. Zugabe von Wasser ad 6000g
16. Einstellung pH=7,0 mit Natriumhydroxid

Während des Abfüllprozesses der so erhaltenen Zusammensetzung in einen Beutel einer Bag-on-Valve Dose werden der Zusammensetzung noch 4,9 Gew.-% kosmetischer Schaumbildner (Treibgas), bezogen auf die treibgashaltige Zusammensetzung, hinzugefügt.

Die vorstehend genannte, beispielhafte Zusammensetzung kann in den Mundstücken manuell durch Aufsprühen als Schaum oder Aerosol in die Mundstücke verteilt werden. Es hat sich gezeigt, dass sich die Zusammensetzung sehr gleichmäßig in den Aussparungen des Mundstücks verteilt.

Es hat sich außerdem gezeigt, dass insbesondere der Putzkörper, aber auch die anderen Aktivstoffe gleichmäßig in dem Schaum oder Aerosol verteilt sind. Damit ist sichergestellt, dass lokale Überdosierungen oder Unterdosierungen vermieden werden. Somit konnten mit der erfindungsgemäßen Zusammensetzung sehr gute Reinigungsergebnisse erzielt werden.

Die beispielhafte Zusammensetzung hat sich außerdem dadurch vorteilhaft ausgezeichnet, dass sie und der aus ihr gebildete Schaum gut und ausreichend lange an dem Mundstuck haftet, um ein Einführen des Mundstücks in die Mundhöhle zu ermöglichen, ohne dass sie oder der Schaum aus dem Mundstuck herausläuft. Das galt insbesondere auch für die Zusammensetzung bzw. den Schaum der sich in der nach unten geöffneten Aussparung für die Aufnahme der Zähne des Unterkiefers, also in Kopfüberlage, befand. Im Mund zerfällt der Schaum aber wieder.

Die Anhaftung (Adhäsion) ist messbar mit einem Tensiometer.

### Beispiel 3: Eignungsprüfung der Zahnpflegezusammensetzung

Um zu überprüfen, ob die erfindungsgemäßen Zusammensetzungen zur Anwendung in einem Mundstück zur gleichzeitigen Reinigung aller Zähne eines Benutzers geeignet sind, wurde eine beispielhafte Zusammensetzung mit einem Glycerin-Gehalt von 25 Gew.-% und einem Wassergehalt von 18 Gew.-% mit einer nicht erfindungsgemäßen Zusammensetzung verglichen, die 8 Gew.-% Glycerin und 9 Gew.-% Wasser enthielt.

Beide Zusammensetzungen wurden aus der Bag-on-Valve Dose in Strangform auf eine Glasplatte appliziert und die Schaumadhäsion und der Zerfall der Schäume über die Zeit (0 Sekunden bis 5 Minuten) untersucht.

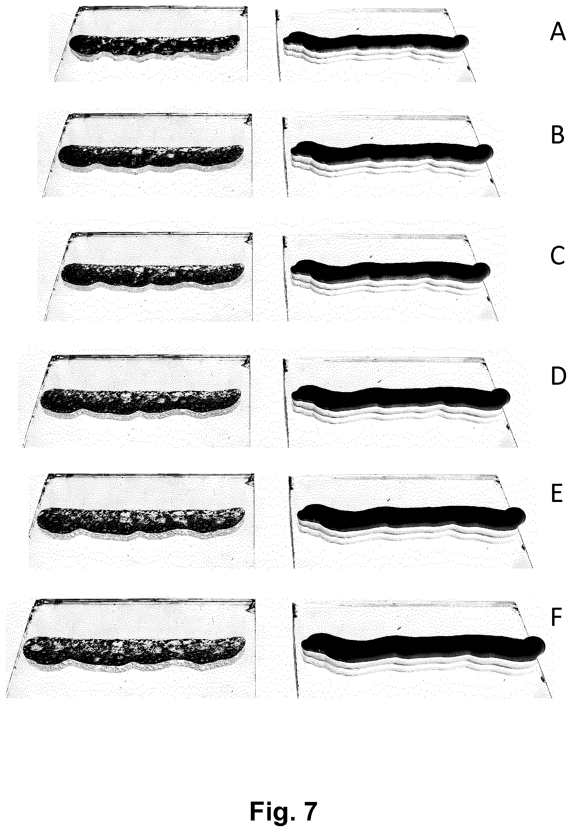

Die Ergebnisse sind in Fig. 7 A-F dargestellt. Der erfindungsgemäße Schaum (links) zeichnet sich durch eine für das Aufbringen der Zusammensetzung auf ein Mundstück ausreichend lange Schaumstabilität aus. Der Schaum zerfällt dann aber wie gewünscht wieder. Im Vergleich dazu schäumt die nicht erfindungsgemäße Zusammensetzung (rechts) sehr stark und der Schaum fällt auch nach längerer Zeit nicht zusammen.

### Beispiel 4: Quantifizierung des Schaumvolumens

Die Zusammensetzungen gemäß dem Beispiel 3 wurden in handelsübliche Kunststoffmesszylinder gefüllt und die prozentuale Abnahme der Menisken (bedingt durch den Schaumzerfall) wurde in Abhängigkeit der Zeit bestimmt.

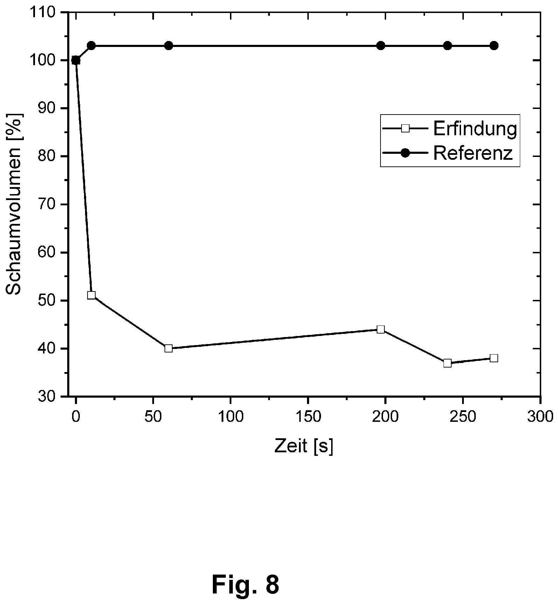

Die Ergebnisse sind in Fig. 8 dargestellt. Es bestätigt sich, dass die erfindungsgemäße Zusammensetzung die gewünschte kurzzeitige Schaumstabilität zeigt, der Schaum dann aber, wie gewünscht, wieder zerfällt.

## Patentansprüche

1. Zahnpflege- und Zahnreinigungszusammensetzung zur Verwendung in einem Verfahren zum gleichzeitigen Reinigen mehrerer, vorzugsweise aller Zähne des Ober- und/oder Unterkiefers, mittels einer hierfür geeigneten Zahnputzvorrichtung, wobei die Zahnpflege- und Zahnreinigungszusammensetzung in aufgeschäumter Form auf einem Mundeinsatz oder mehreren, vorzugsweise zwei, Mundeinsätzen der Zahnputzvorrichtung dargereicht wird, und wobei die Zahnpflege- und Zahnreinigungszusammensetzung als wesentliche Komponenten umfasst:
- 10 bis 40 Gew.-%, vorzugsweise 15 bis 30 Gew.-%, Glycerin,
- 10 bis 23,5 Gew.-% eines gefällten Silikats oder mehrerer gefällter Silikate, vorzugsweise einer Mischung aus 2 bis 4 gefällten Silikaten,
- 0,05 bis 0,5 Gew.-%, vorzugsweise 0,05 bis 0,2 Gew.-%, eines Viskositätsverstärkers oder mehrerer Viskositätsverstärker,
- eine oberflächenaktiven Substanz oder mehrerer oberflächenaktiven Substanzen, vorzugsweise mit einem Anteil von 0,2 bis 4,0 Gew.-%, noch mehr bevorzugt mit einem Anteil von 0,2 bis 2,5 Gew.-%, der einen oder der mehreren oberflächenaktiven Substanz(en), und
- mindestens 10 Gew.-% Wasser.

2. Zahnpflege- und Zahnreinigungszusammensetzung zur Verwendung nach Anspruch 1 in einem Verfahren zur Vorbeugung und/oder Behandlung von Zahn- und Zahnfleischerkrankungen, insbesondere von Karies, Parodontose und/oder Plaque.

3. Zahnpflege- und Zahnreinigungszusammensetzung, umfassend
- 15 bis 30 Gew.-%, vorzugsweise mindestens 20 Gew.-%, Glycerin,
- optional wenigstens ein weiteres Feuchthaltemittel, neben Glycerin, wobei das wenigstens eine weitere Feuchthaltemittel vorzugsweise Sorbitol oder hydrogeniertes Stärkehydrolysat, das vorzugsweise einen Sorbitol-Anteil im Bereich von 65 bis 75 Gew.-% aufweist, ist oder umfasst,
- 10 bis 23,5 Gew.-% eines Gemisches aus zwei oder mehr, vorzugsweise zwei bis vier, gefällten Silikaten,
- 0,2 bis 4,0 Gew.-%, vorzugsweise 0,2 bis 2,5 Gew.-%, einer oberflächenaktiven Substanz oder mehrerer oberflächenaktiven Substanzen,
- 0,05 bis 0,5 Gew.-% , vorzugsweise 0,05 bis 0,2 Gew.-%, eines Viskositätsverstärkers oder mehrerer Viskositätsverstärker,
- optional wenigstens einen Wirkstoff und/oder wenigstens einen geeigneten Hilfs- oder Zusatzstoff, und
- mindestens 10 Gew.-% Wasser, wobei
das Gemisch aus zwei oder mehr gefällten Silikaten mindestens ein gefälltes Silikat mit einer mittleren Teilchengröße in einem Bereich von 7 bis 8,5 µm und einer Einlehner Abrasion im Bereich von mehr als 5,0 bis 25 mg Verlust / 100 000 Umdrehungen, mindestens ein gefälltes Silikat mit einer mittleren Teilchengröße im Bereich von mehr als 8,5 bis 10 µm und einer Einlehner Abrasion im Bereich von mehr als 2,5 bis 5,0 mg Verlust / 100 000 Umdrehungen, und mindestens ein gefälltes Silikat mit einer mittleren Teilchengröße im Bereich von mehr als 10 bis 14 µm und einer Einlehner Abrasion im Bereich von weniger als 2,5 Verlust / 100 000 Umdrehungen umfasst,
die Zusammensetzung kein Polyethylenglykol enthält oder der Anteil an Polyethylenglycol in der Zusammensetzung maximal 0,8 Gew.-% beträgt, und
die Zusammensetzung als Viskositätsverstärker Xanthan Gum umfasst oder der Viskositätsverstärker Xanthan Gum ist, und der Anteil an Cellulose oder Cellulose-Derivaten und/oder Algenpolysacchariden, insbesondere Carragenaan, in der Zusammensetzung im Bereich von 0 bis maximal 0,1 Gew.-% liegt.

4. Zahnpflege- und Zahnreinigungszusammensetzung nach Anspruch 3, wobei die Zusammensetzung als weiteres Feuchthaltemittel hydrogeniertes Stärkehydrolysat, das vorzugsweise einen Sorbitol-Anteil im Bereich von 65 bis 75 Gew.-% aufweist, oder Sorbitol umfasst oder das weitere Feuchthaltemittel hydrogeniertes Stärkehydrolysat, das vorzugsweise einen Sorbitol-Anteil im Bereich von 65 bis75 Gew.-% aufweist, oder Sorbitol ist.

5. Zahnpflege- und Zahnreinigungszusammensetzung nach Anspruch 4, wobei die Zusammensetzung 16,25 bis 30 Gew.-% Sorbitol umfasst.

6. Zusammensetzung nach einem der Ansprüche 3 bis 5, wobei die Zusammensetzung eine Viskosität im Bereich von 200 bis 30.000 m Pa s, vorzugsweise von weniger als 10.000 mPa s, noch mehr bevorzugt in einem Bereich von 500 bis 9.000 mPa s, jeweils gemessen mit einem Brookfield Viskosimeter, vorzugsweise einem Rotationsviskosimeter DV2T der Firma Brookfield mit Heliopath T-Spindeln vom Spindeltyp C, bei einer Drehzahl von 10 rpm, 20 °C und 1013,25 mbar, aufweist.

7. Zusammensetzung nach einem der vorhergehenden Ansprüche 3 bis 6, wobei die Zusammensetzung kein Polyethylenglykol enthält oder der Anteil an Polyethylenglycol in der Zusammensetzung maximal 0,4 Gew.-%, beträgt.

8. Zusammensetzung nach einem der vorhergehenden Ansprüche 3 bis 7, wobei der Anteil an Cellulose oder Cellulose-Derivaten und/oder Algenpolysacchariden, insbesondere Carragenaan, in der Zusammensetzung im Bereich von 0 bis 0,05 Gew.-%, liegt.

9. Zusammensetzung nach einem der vorhergehenden Ansprüche 3 bis 8, wobei die Zusammensetzung weiterhin wenigstens einen Wirkstoff umfasst, der vorzugsweise ausgewählt ist aus der Gruppe, bestehend aus einer oder mehrerer Verbindungen zur Remineralisation oder Keramisierung wie z.B. Hydroxylapatit, einem oder mehrerer Fluoridwirkstoffe, einem Mittel zur Zahnfleischpflege, einem desinfizierenden Bestandteil und einer beliebigen Mischung davon, wobei der Anteil des einen oder der mehreren Wirkstoffe an der Zusammensetzung vorzugsweise 0,5 bis 8 Gew.-% beträgt.

10. Zusammensetzung nach einem der vorhergehenden Ansprüche 3 bis 9, wobei die Zusammensetzung umfasst oder besteht aus:
- 16,25 bis 30 Gew.-% Sorbitol,
- 15 bis 30 Gew.-%, Glycerin
- 1 bis 2 Gew.-% aminfluoridhaltiger Rohstoff mit 20-50% Wirkstoffanteil
- 6 bis 13 Gew.%, vorzugsweise 7 bis 13 Gew.-%, gefälltes Silikat der Kategorie 1
- 3 bis 7 Gew.-%, gefälltes Silikat der Kategorie 2
- 1 bis 3,5 Gew.-%, vorzugsweise 1,5 bis 3 Gew.-%, gefälltes Silikat der Kategorie 3
- 0,1-2 Gew.-%, vorzugsweise 0,2-1 Gew.-%, Natriumlaurylsulfat
- 0,1-2 Gew.-%, vorzugsweise 0,15-1 Gew.-%, Cocamidopropylbetain
- 0,05-0,2 Gew.-% Xanthan Gum
- sowie mindestens 10 Gew.-% Wasser.

11. Zusammensetzung nach einem der vorhergehenden Ansprüche 3 bis 10, wobei die Zusammensetzung übliche Hilfs- und Zusatzstoffe, vorzugsweise ausgewählt aus der Gruppe, bestehend aus Aromastoffe, Süßungsmittel, Konservierungsmittel, Pigmente, Farbstoffe, Puffersubstanzen und einer beliebigen Mischung davon, umfasst, wobei der Anteil des einen oder der mehreren Hilfs- und Zusatzstoffe an der Zusammensetzung vorzugsweise 0,1 bis 5 Gew.-% beträgt.

12. Zusammensetzung nach einem der vorhergehenden Ansprüche 3 bis 11, wobei die Zusammensetzung als Schaum oder Aerosol vorliegt.

13. System, bestehend aus einer Zahnpflege- und Zahnreinigungszusammensetzung nach einem der vorhergehenden Ansprüche 3 bis 11 und einem Spender mit Mitteln zur Bevorratung, zur Förderung und Abgabe der Zahnpflege- und Zahnreinigungszusammensetzung,
wobei das Mittel zur Abgabe der Zusammensetzung ein Auslassventil mit einer Auslassöffnung aufweist, und
wobei die Zusammensetzung und der Spender derart zusammenwirken, dass bei Betätigung des Auslassventils das Zahnreinigungsmittel in strangförmiger Form aus der Auslassöffnung austritt.

14. System nach Anspruch 13, wobei das Mittel zur Förderung die Zusammensetzung mittels eines Treibgases oder Treibgasgemisches befördert und der Spender ausreichend druckstabil gegenüber dem Expansionsdruck der treibgashaltigen Zusammensetzung oder des Treibgases oder Treibgasgemisches ausgebildet sind.

15. System nach Anspruch 13 oder Anspruch 14, wobei das Mittel zur Bevorratung und Angabe der Zusammensetzung eine Aerosoldose oder eine Bag-on-Valve Dose ist.

16. System nach einem der Ansprüche 13 bis 15, wobei die Zahnpflege- und Zahnreinigungszusammensetzung in Kombination mit einem Treibgas oder Treibgasgemisch vorliegt, insbesondere wobei das Zahnpflege- und Zahnreinigungsmittel ein Treibgas oder Treibgasgemisch enthält, das eine komprimierbare Flüssigkeit ist, die bei Normaldruck einen Siedepunkt von -10 °C oder höher aufweist, oder wobei das Treibgas oder Treibgasgemisch keine komprimierbare Flüssigkeit, vorzugsweise Distickstoffoxid, ist.

17. System nach einem der Ansprüche 12 bis 16, wobei der Treibgasgehalt der Zusammensetzung 0 bis 15 Gew.-%, vorzugsweise kleiner gleich 12 Gew.-%, beträgt, oder im Fall einer nicht komprimierbaren Flüssigkeit, vorzugsweise von Distickstoffoxid, der resultierende Druck im Bereich von 6 bis 10 bar liegt.

18. Zahnpflege- und Zahnreinigungszusammensetzung, umfassend 10 bis 40 Gew.-%, vorzugsweise 15 bis 30 Gew.-%, Glycerin, 10 bis 23,5 Gew.-% eines gefällten Silikats oder mehrerer gefällter Silikate, vorzugsweise einer Mischung aus 2 bis 4 gefällten Silikaten, 0,05 bis 0,5 Gew.-% eines Viskositätsverstärkers oder mehrerer Viskositätsverstärker, eine oberflächenaktive Substanz oder mehrere oberflächenaktive Substanzen, vorzugsweise mit einem Anteil von 0,2 bis 4,0 Gew.-% der einen oder der mehreren oberflächenaktiven Substanz(en), und mindestens 10 Gew.-% Wasser, zur Verwendung in einem Verfahren zum gleichzeitigen Reinigen mehrerer, vorzugsweise aller Zähne eines Subjekts, wobei das Zahnreinigungsverfahren die folgenden Schritte umfasst:
- Bereitstellen der Zahnpflege- und Zahnreinigungszusammensetzung,
- Auftragen/Aufbringen der Zusammensetzung auf einen Mundeinsatz oder mehrere Mundeinsätze einer Vibrationszahnputzvorrichtung für die gleichzeitige Reinigung mehrerer, vorzugsweise aller Zähne eines Benutzers, der bzw. die für die Reinigung mehrerer, bevorzugt aller Zähne eines Benutzers geeignet ist/sind,
- Einführen des Mundeinsatzes bzw. der Mundeinsätze mit der Zusammensetzung in den Mund des Benutzers und
- Reinigen mehrerer, bevorzugt aller Zähne des Benutzers mit dem Mundeinsatz oder den Mundeinsätzen und der Zusammensetzung durch Aktivieren der Vibrationszahnputzvorrichtung.

19. Zahnpflege- und Zahnreinigungszusammensetzung zur Verwendung in einem Verfahren nach Anspruch 18, wobei die bereitgestellte Zahnpflege- und Zahnreinigungszusammensetzung eine Zusammensetzung gemäß einem der Ansprüche 3 bis 14 ist.

20. Zahnpflege- und Zahnreinigungszusammensetzung zur Verwendung nach Anspruch 1 oder Anspruch 2, wobei die Zahnpflege- und Zahnreinigungszusammensetzung eine Zusammensetzung gemäß einem der Ansprüche 3 bis 12 ist.

21. Zahnpflege- und Zahnreinigungszusammensetzung nach einem der Ansprüche 3 bis 12 zur Verwendung in einem Verfahren zur Vorbeugung und/oder Behandlung von Zahn- und Zahnfleischerkrankungen, insbesondere von Karies, Parodontose und Plaque.

## Claims

1. A dental care- and dental cleaning composition for use in a method of simultaneously cleaning a plurality of, preferably all, teeth of the upper and/or lower jaw by means of a dental brushing device suitable therefor, wherein the dental care- and dental cleaning composition is presented in foamed form on one or a plurality of, preferably two mouth inserts of the dental brushing device, and wherein the dental care- and dental cleaning composition comprises as essential components:
- 10 to 40 wt.-%, preferably 15 to 30 wt.-%, glycerol,
- 10 to 23.5 wt.-% of one precipitated silicate, or a plurality of precipitated silicates, preferably a mixture of 2 to 4 precipitated silicates,
- 0.05 to 0.5wt.-%, preferably 0.05 to 0.2 wt.-% , of one or more viscosity amplifier(s),
- one or more surfactant(s), preferably in a proportion of 0.2 to 4.0 wt.-% , more preferably in a proportion of 0.2 to 2.5 wt.-%, of the one or more surfactant(s), and
- at least 10 wt.-% of water.

2. Dental care- and dental cleaning composition for use according to claim 1 in a method for prevention and/or treatment of dental and gum diseases, in particular caries, periodontitis and/or plaque.

3. Dental care- and dental cleaning composition comprising
- 15 to 30 wt.-% , preferably at least 20 wt.-% , of glycerol,
- optionally at least one further humectant, besides glycerol, wherein the at least one further humectant is or comprises preferably sorbitol or hydrogenated starch hydrolysate, preferably having a sorbitol proportion in the range of 65 to 75 wt.-%,
- 10 to 23.5 wt.-% of a mixtures of two or more, preferably two to four precipitated silicates,
- 0.2 to 4.0 wt.-%, preferably 0.2 to 2.5wt.-%, of one or more surfactants,
- 0.05 to 0.5wt.-%, preferably 0.05 to 0.2wt.-%, of one or more viscosity amplifiers,
- optionally at least one active substance and/or at least one suitable excipient or additive, and
- at least 10wt.-% of water, wherein
the mixture of two or more precipitated silicates comprises at least one precipitated silicate having an average particle size in a range of 7 to 8.5µm and an Einlehner-abrasion in a range of 5 to 25 mg loss / 100 000 revolutions, at least one precipitated silicate having an average particle size in a range of more than 8.5 µm to 10 µm and an Einlehner-abrasion in a range of 2.5 to 5.0 mg loss / 100 000 revolutions, and at least a precipitated silicate having an average particle size in a range of more than 10 to 14 µm and an Einlehner-abrasion in the range of less than 2.5 loss / 100 000 revolutions,
the composition does not include polyethylene glycol or the proportion of polyethylene glycol in the composition is at most 0.8 wt.-%, and
the composition comprises as viscosity amplifier Xanthan Gum or the viscosity amplifier is Xanthan Gum, and the proportion of Cellulose or Cellulose derivates and/or algal polysaccharides, in particular Carrageenan, in the composition is in the range of 0 to at most 0.1 wt.-%.

4. Dental care- and dental cleaning composition according to claim 3, wherein the composition comprises as further humectant hydrogenated starch hydrolysate, preferably having a sorbitol proportion in the range of 65 to 75wt%, or sorbitol, or the further humectant is hydrogenated starch hydrolysate, preferably having a sorbitol proportion in the range of 65 to 75wt%, or sorbitol.

5. Dental care- and dental cleaning composition according to claim 4, wherein the composition comprises 16.25 to 30wt.-% of sorbitol.

6. Composition according to any one of claims 3 to 5, wherein the composition comprises a viscosity in the range of 200 to 30,000 mPa s, preferably less than 10,000 mPa s, more preferably in a range of 500 to 9,000 mPa s, each measured with a Brookfield viscometer, preferably a Brookfield DV2T rotational viscometer with Heliopath T-spindles of spindle type C, at a rotational speed of 10 rpm, 20 °C and 1013.25 mbar.

7. The composition according to any one of the preceding claims 3 to 6, wherein the composition does not include polyethylene glycol or the proportion of polyethylene glycol in the composition is at most 0.4wt.-%.

8. The composition according to any one of the preceding claims 3 to 7, wherein the proportion of cellulose or cellulose derivatives and/or algal polysaccharides, in particular Carragenaan, in the composition is in the range of 0 to 0.05wt.-%.

9. The composition according to any one of the preceding claims 3 to 8, wherein the composition further comprises at least one active substance preferably selected from the group consisting of one or more compounds for remineralisation or ceramization such as hydroxyapatite, one or more fluoride active substances, a gum care agent, a disinfectant component and any mixture thereof, wherein the proportion of the one or more active substances in the composition is preferably 0.5 to 8wt.-%.

10. The composition according to any one of the preceding claims 3 to 9, wherein the composition comprises or consists of:
- 16.25 to 30 wt.-% sorbitol,
- 15 to 30 wt.-%, glycerol
- 1 to 2wt.-% of amine fluoride-containing raw material with a proportion of 20-50% active substance
- 6 to 13 wt.-%, preferably 7 to 13 wt.%, precipitated silicate of category 1
- 3 to 7 wt.-%, precipitated silicate of category 2
- 1 to 3.5 wt.-%, preferably 1.5 to 3 wt.-%, precipitated silicate of category 3
- 0.1-2 wt.-%, preferably 0.2-1 wt.-%, sodium lauryl sulphate
- 0.1-2 wt.-%, preferably 0.15-1 wt.-%, cocamidopropyl betaine
- 0.05-0.2 wt.-% Xanthan Gum
- and at least 10wt.-% of water.

11. The composition according to any one of the preceding claims 3 to 10, wherein the composition comprises usual excipients and additives, preferably selected from the group consisting of flavourings, sweeteners, conserving agents, pigments, colourants, buffer substances and any mixture thereof, wherein the proportion of the one or more excipients and additives in the composition is preferably 0.1-5wt.-%.

12. The composition according to any one of the preceding claims 3 to 11, wherein the composition is in the form of a foam or an aerosol.

13. A system comprising a dental care and dental cleaning composition according to any one of the preceding claims 3 to 11 and a dispenser having means for storing, conveying and dispensing the dental care and dental cleaning composition,
wherein the means for dispensing the composition comprises an outlet valve having an outlet opening, and
wherein the composition and the dispenser cooperate such that upon actuation of the outlet valve, the dental cleaning composition exits the outlet opening in a stranded form.

14. The system of claim 13, wherein the means for conveying convey the composition by means of a propellant gas or propellant gas mixture, and the dispenser are formed to be sufficiently pressure stable with respect to the expansion pressure of the propellant gas-containing composition or the propellant gas or propellant gas mixture.

15. The system according to claim 13 or claim 14, wherein the means for storing and dispensing the composition is an aerosol can or a bag-on-valve can.

16. The system according to any one of claims 13 to 15, wherein the dental care and dental cleaning composition is provided in combination with a propellant gas or propellant gas mixture, in particular wherein the dental care and dental cleaning composition comprises a propellant gas or propellant gas mixture which is a compressible liquid having a boiling point of -10 °C or higher at normal pressure, or wherein the propellant gas or propellant gas mixture is not a compressible liquid, preferably dinitrogen oxide.

17. The system according to any one of claims 12 to 16, wherein the propellant gas proportion of the composition is 0 to 15wt.-%, preferably less than or equal to 12wt.-%, or in the case of a non-compressible liquid, preferably dinitrogen oxide, the resulting pressure is in the range of 6 to 10 bar.

18. Dental care and dental cleaning composition, comprising:
- 10 to 40 wt.-%, preferably 15 to 30 wt.-%, glycerol, 10 to 23.5 wt.-% of one or more precipitated silicates, preferably a mixture of 2 to 4 precipitated silicates, 0.05 to 0.5 wt.-% of one or more viscosity amplifiers, one or more surfactants, preferably with an Proportion of 0.2 to 4.0 wt.-% of the one or the more surfactant(s), and at least 10 wt.-% water, for use in a method of simultaneously cleaning a plurality, preferably all teeth of a subject, wherein the dental cleaning method comprises the following steps:
- providing the dental care and dental cleaning composition,
- coating/applying the composition to one or more mouth inserts of a vibratory dental brushing device for the simultaneously cleaning of a plurality, preferably all, teeth of a user suitable for the cleaning of a plurality, preferably all, teeth of a user,
- inserting the mouth insert or mouth inserts, respectively with the composition into the mouth of the user, and
- cleaning a plurality, preferably all teeth of the user with the one or more mouth inserts and the composition by activating the vibratory dental brushing device.

19. The dental care and dental cleaning composition for use in a method according to claim 18, wherein the provided dental care and dental cleaning composition is a composition according to any one of claims 3 to 14.

20. The dental care and cleaning composition for use according to claim 1 or claim 2, wherein the dental care and dental cleaning composition is a composition according to any one of claims 3 to 12.

21. The dental care- and dental cleaning composition according to any one of claims 3 to 12 for use in a method of prevention and/or treatment of dental and gum diseases, in particular caries, periodontitis and plaque.

## Revendications

1. Composition de soin et de nettoyage dentaire, destinée à être utilisée dans un procédé de nettoyage simultané de plusieurs, de préférence de toutes les dents des mâchoires supérieure et/ou inférieure, au moyen d'un dispositif de nettoyage dentaire adapté à cet effet, la composition de soin et de nettoyage dentaire étant administrée sous forme expansée sur un insert buccal ou plus, de préférence sur deux inserts buccaux du dispositif de nettoyage dentaire et la composition de soin et de nettoyage dentaire comprenant en tant que composants majeurs :
- de 10 à 40 % en poids, de préférence de 15 à 30 % en poids, de glycérine,
- de 10 à 23,5 % en poids d'un silicate précipité ou de plusieurs silicates précipités, de préférence d'un mélange de 2 à 4 silicates précipités,
- de 0,05 à 0,5 % en poids, de préférence de 0,05 à 0,2 % en poids d'un renforçateur de viscosité ou de plusieurs renforçateurs de viscosité,
- une substance à effet tensioactif ou plusieurs substances à effet tensioactif, de préférence à raison d'une part de 0,2 à 4,0 % en poids, de manière encore plus préférentielle, d'une part de 0,2 à 2,5 % en poids, de l'une ou des plusieurs substance(s) à effet tensioactif, et
- au moins 10 % en poids d'eau.

2. Composition de soin et de nettoyage dentaire, destinée à l'utilisation selon la revendication 1 dans un procédé visant à prévenir et/ou à traiter des affections dentaires et gingivales, notamment des caries, des parodontoses et/ou la plaque dentaire.

3. Composition de soin et de nettoyage dentaire, comprenant :
- de 15 à 30 % en poids, de préférence au moins 20 % en poids de glycérine,
- en option, au moins un autre agent humectant, conjointement à la glycérine, l'au moins un autre agent humectant étant ou comprenant de préférence du sorbitol ou un hydrolysat d'amidon hydrogéné, qui comporte de préférence une part de sorbitol de l'ordre de 65 à 75 % en poids,
- de 10 à 23,5 % en poids d'un mélange de deux ou plus, de préférence de deux à quatre silicates précipités,
- de 0,2 à 4,0 % en poids, de préférence de 0,2 à 2,5 % en poids d'une substance à effet tensioactif ou de plusieurs substances à effet tensioactif,
- de 0,05 à 0,5 % en poids, de préférence de 0,05 à 0,2 % en poids d'un renforçateur de viscosité ou de plusieurs renforçateurs de viscosité,
- en option, au moins un principe actif et/ou au moins un excipient ou additif adapté, et
- au moins 10 % en poids d'eau,
le mélange de deux silicates précipités ou plus comprenant au moins un silicate précipité d'une taille moyenne des particules dans un ordre de 7 à 8,5 µm et faisant preuve d'un indice d'abrasion d'Einlehner de l'ordre de plus de 5,0 à 25 mg de perte / 100 000 tours, au moins un silicate précipité d'une taille moyenne des particules de l'ordre de plus de 8,5 à 10 µm et faisant preuve d'un indice d'abrasion d'Einlehner de l'ordre de plus de 2,5 à 5,0 mg de perte / 100 000 tours, et au moins un silicate précipité d'une taille moyenne des particules de l'ordre de plus de 10 à 14 µm et faisant preuve d'un indice d'abrasion d'Einlehner de l'ordre de moins de 2,5 mg de perte / 100 000 tours,
la composition ne contenant aucun polyéthylène glycol ou la part de polyéthylène glycol dans la composition s'élevant au maximum à 0,8 % en poids, et
la composition comprenant en tant que renforçateur de viscosité de la gomme de xanthane ou le renforçateur de viscosité étant de la gomme de xanthane, et la part de cellulose ou de dérivés cellulosiques et/ou de polysaccharides d'algues, notamment de carraghénane dans la composition se situant dans l'ordre de 0 à un maximum de 0,1 % en poids.

4. Composition de soin et de nettoyage dentaire selon la revendication 3, la composition comprenant en tant qu'autre agent humectant un hydrolysat d'amidon hydrogéné, qui comporte de préférence une part de sorbitol de l'ordre de 65 à 75 % en poids, ou du sorbitol ou l'autre agent humectant étant un hydrolysat d'amidon hydrogéné, qui comporte de préférence une part de sorbitol de l'ordre de 65 à 75 % en poids ou qui est du sorbitol.

5. Composition de soin et de nettoyage dentaire selon la revendication 4, la composition comprenant de 16,25 à 30 % en poids de sorbitol.

6. Composition selon l'une quelconque des revendications 3 à 5, la composition faisant preuve d'une viscosité de l'ordre de 200 à 30.000 mPas, de préférence de moins de 10.000 mPas, de manière encore plus préférentielle, dans un ordre de 500 à 9.000 mPas, mesurée chaque fois à l'aide d'un viscosimètre Brookfield, de préférence d'un viscosimètre rotatif DV2T de la société Brookfield, pourvu de mobiles en forme de T de type C, à une vitesse de rotation de 10 rpm, à 20 °C et à 1013,25 mbar.

7. Composition selon l'une quelconque des revendications précédentes 3 à 6, la composition ne contenant pas de polyéthylène glycol ou la part de polyéthylène glycol dans la composition s'élevant au maximum à 0,4 % en poids.

8. Composition selon l'une quelconque des revendications précédentes 3 à 7, la part de cellulose ou de dérivés cellulosiques et/ ou de polysaccharides d'algues, notamment de carraghénane dans la composition se situant dans l'ordre de 0 à 0,05 % en poids.

9. Composition selon l'une quelconque des revendications précédentes 3 à 8, la composition comprenant par ailleurs au moins un principe actif, qui est sélectionné de préférence dans le groupe consistant dans un ou dans plusieurs composés de reminéralisation ou de céramisation, comme par ex. l'hydroxyapatite, un ou plusieurs principes actifs fluorés, un produit de soin gingival, un élément désinfectant et un mélange quelconque des ceux-ci, la part de l'un ou des plusieurs principes actifs dans la composition s'élevant de préférence à de 0,5 à 8 % en poids.

10. Composition selon l'une quelconque des revendications précédentes 3 à 9, la composition comprenant ou consistant dans :
- de 16,25 à 30 % en poids de sorbitol,
- de 15 à 30 % en poids de glycérine
- de 1 à 2 % en poids de matière première au fluorure d'amine, avec une part de 20 à 50 % de principe actif,
- de 6 à 13 % en poids, de préférence de 7 à 13 % en poids d'un silicate précipité, de la catégorie 1,
- de 3 à 7 % en poids d'un silicate précipité, de la catégorie 2,
- de 1 à 3,5 % en poids, de préférence de 1,5 à 3 % en poids d'un silicate précipité, de la catégorie 3,
- de 0,1 à 2 % en poids, de préférence de 0,2 à 1 % en poids, de laurylsulfate de sodium,
- de 0,1 à 2 % en poids, de préférence de 0,15 à 1 % en poids de bétaïne de cocamidopropyle
- de 0,05 à 0,2 % en poids de gomme de xanthane,
- ainsi qu'au moins 10 % en poids d'eau.

11. Composition selon l'une quelconque des revendications précédentes 3 à 10, la composition comprenant des excipients et des adjuvants usuels, sélectionnés de préférence dans le groupe consistant dans les agents aromatiques, les édulcorants, les conservateurs, les pigments, les colorants, les substances tampons et un mélange quelconque de ceux-ci, la part de l'un ou des plusieurs excipients et adjuvants dans la composition s'élevant de préférence à de 0,1 à 5 % en poids.

12. Composition selon l'une quelconque des revendications précédentes 3 à 11, la composition se présentant sous la forme d'une mousse ou d'un aérosol.

13. Système, consistant dans une composition de soin et de nettoyage dentaire selon l'une quelconque des revendications précédentes 3 à 11 et d'un distributeur pourvu de moyens de stockage, de transport et de distribution de la composition de soin et de nettoyage dentaire,
le moyen de distribution de la composition comportant une soupape d'échappement, dotée d'un orifice d'échappement, et
la composition et le distributeur coopérant de telle sorte que lors de l'actionnement de la soupape d'échappement, le produit nettoyant dentaire sorte de l'orifice d'échappement sous la forme d'un filament.

14. System selon la revendication 13, le moyen de transport transportant la composition à l'aide d'un gaz propulseur ou d'un mélange de gaz propulseurs et le distributeur étant conçu pour être suffisamment stable à la pression d'expansion de la composition contenant un gaz propulseur ou du gaz propulseur ou du mélange de gaz propulseur.

15. Système selon la revendication 13 ou la revendication 14, le moyen de stockage et de distribution de la composition étant une bombe aérosol ou Bag-on-Valve.

16. Système selon l'une quelconque des revendications 13 à 15, la composition de soin et de nettoyage dentaire se présentant en association avec un gaz propulseur ou un mélange de gaz propulseurs, notamment le produit de soin et de nettoyage dentaire contenant un gaz propulseur ou un mélange de gaz propulseurs qui est un liquide comprimable, qui à une pression normale fait preuve d'un point d'ébullition de -10 °C ou plus élevé, ou le gaz propulseur ou le mélange de gaz propulseurs n'étant pas un liquide comprimable, de préférence un oxyde nitreux.

17. Système selon l'une quelconque des revendications 12 à 16, la teneur en gaz propulseur de la composition s'élevant à de 0 à 15 % en poids, de préférence étant inférieure ou égale à 12 % en poids, ou dans le cas d'un liquide non comprimable, de préférence d'un oxyde nitreux, la pression résultante se situant dans l'ordre de 6 à 10 bar.

18. Composition de soin et de nettoyage dentaire, comprenant de 10 à 40 % en poids, de préférence de 15 à 30 % en poids de glycérine, de 10 à 23,5 % en poids d'un silicate précipité ou de plusieurs silicates précipités, de préférence d'un mélange de 2 à 4 silicates précipités, de 0,05 à 0,5 % en poids d'un renforçateur de viscosité ou de plusieurs renforçateurs de viscosité, une substance à effet tensioactif ou plusieurs substances à effet tensioactif, de préférence à raison d'une part de 0,2 à 4,0 % en poids de l'une ou des plusieurs substance(s) à effet tensioactif, et au moins 10 % en poids d'eau, destinée à être utilisée dans un procédé de nettoyage simultané de plusieurs, de préférence de toutes les dents d'un sujet, le procédé de nettoyage des dents comprenant les étapes suivantes, consistant à :
- mettre à disposition la composition de soin et de nettoyage dentaire,
- appliquer/déposer la composition sur un insert buccal ou sur plusieurs inserts buccaux d'un dispositif de nettoyage dentaire vibratoire pour le nettoyage simultané de plusieurs, de préférence de toutes les dents d'un utilisateur, qui est ou qui sont adapté(s) pour le nettoyage de plusieurs, de préférence de toutes les dents d'un utilisateur,
- introduire l'insert buccal ou les inserts buccaux pourvu(s) de la composition dans la bouche de l'utilisateur, et
- nettoyer plusieurs, de préférences toutes les dents de l'utilisateur avec l'insert buccal ou les inserts buccaux et la composition, par activation du dispositif de nettoyage dentaire vibratoire.

19. Composition de soin et de nettoyage dentaire, destinée à l'utilisation dans un procédé selon la revendication 18, la composition de soin et de nettoyage dentaire mise à disposition étant une composition selon l'une quelconque des revendications 3 à 14.

20. Composition de soin et de nettoyage dentaire, destinée à l'utilisation selon la revendication 1 ou la revendication 2, la composition de soin et de nettoyage dentaire étant une composition selon l'une quelconque des revendications 3 à 12.

21. Composition de soin et de nettoyage dentaire selon l'une quelconque des revendications 3 à 12, destinée à l'utilisation dans un procédé visant à prévenir et/ou à traiter des affections dentaires et gingivales, notamment des caries, des parodontoses et/ou la plaque dentaire
